# EUROPEAN PATENT SPECIFICATION

(11) **EP 3 060 669 B1**
(45) Date of publication and mention of the grant of the patent: **11.12.2024**
(21) Application number: 14796300.3
(22) Date of filing: 24.10.2014
(51) Int. Cl.: C12N 15/86

(54) **AAV-5 PSEUDOTYPED VECTOR FOR GENE THERAPY FOR NEUROLOGICAL DISEASES**
ADENOASSOZIIERTER VIRUS MIT AAV-5-HÜLLPROTEINEN FÈR GENTHERAPIE VON NEUROLOGISCHEN ERKRANKUNGEN
VECTEUR ADÉNOVIRAL-ASSOCIÉ PSEUDOTYPÉ AAV-5 POUR LA THERAPIE GÉNIQUE DE MALADIES NEUROLOGIQUES

(30) Priority: 24.10.2013 EP 13190141
(43) Date of publication of application: 31.08.2016
(73) Proprietor: uniQure IP B.V., 1105 BP Amsterdam (NL)
(72) Inventor: BLITS, Bas, NL-1105 BP Amsterdam (NL)
(74) Representative: Nederlandsch Octrooibureau
(86) International application number: PCT/NL2014/050737
(87) International publication number: WO 2015/060722

(56) References cited:
- US-A1- 2004 258 671
- US-A1- 2013 225 666
- N D FAGOE ET AL: "A compact dual promoter adeno-associated viral vector for efficient delivery of two genes to dorsal root ganglion neurons", GENE THERAPY, vol. 21, no. 3, 28 November 2013 (2013-11-28), pages 242 - 252, XP055165690, ISSN: 0969-7128, DOI: 10.1038/gt.2013.71
- A. S. REDDY ET AL: "Bone Marrow Transplantation Augments the Effect of Brain- and Spinal Cord-Directed Adeno-Associated Virus 2/5 Gene Therapy by Altering Inflammation in the Murine Model of Globoid-Cell Leukodystrophy", JOURNAL OF NEUROSCIENCE, vol. 31, no. 27, 6 July 2011 (2011-07-06), pages 9945 - 9957, XP055099621, ISSN: 0270-6474, DOI: 10.1523/JNEUROSCI.1802-11.2011
- LIN ET AL: "AAV2/5 vector expressing galactocerebrosidase ameliorates CNS disease in the murine model of globoid-cell leukodystrophy more efficiently than AAV2", MOLECULAR THERAPY, SEPTEMBER 2005, NATURE PUBLISHING GROUP, GB, vol. 12, no. 3, 29 June 2005 (2005-06-29), pages 422 - 430, XP005472376, ISSN: 1525-0016, DOI: 10.1016/J.YMTHE.2005.04.019
- LUCY VULCHANOVA ET AL: "Differential adeno-associated virus mediated gene transfer to sensory neurons following intrathecal delivery by direct lumbar puncture", MOLECULAR PAIN,, vol. 6, no. 1, 28 May 2010 (2010-05-28), pages 31 - 39, XP055099564, ISSN: 1744-8069, DOI: 10.1186/1744-8069-6-31
- G WATSON ET AL: "Intrathecal administration of AAV vectors for the treatment of lysosomal storage in the brains of MPS I mice", GENE THERAPY, JUNE 2006, vol. 13, no. 11, 16 February 2006 (2006-02-16), pages 917 - 925, XP055074794, ISSN: 0969-7128, DOI: 10.1038/sj.gt.3302735
- STEVEN JAMES GRAY: "Gene therapy and neurodevelopmental disorders", NEUROPHARMACOLOGY,JUNE 2006, vol. 68, 27 June 2012 (2012-06-27), pages 136 - 142, XP055099736, ISSN: 0028-3908, DOI: 10.1016/j.neuropharm.2012.06.024
- TENENBAUM L ET AL: "Recombinant AAV-mediated gene delivery to the central nervous system", JOURNAL OF GENE MEDICINE, JOHN WILEY & SONS, INC, US, vol. 6 Suppl 1, 10 February 2004 (2004-02-10), pages S212 - S222, XP002715192, ISSN: 1099-498X, DOI: 10.1002/JGM.506
- S J GRAY ET AL: "Global CNS gene delivery and evasion of anti-AAV-neutralizing antibodies by intrathecal AAV administration in non-human primates", GENE THERAPY, vol. 20AAv9//AAV2/5 cisterna magna ou lumber sister, no. 4, 10 January 2013 (2013-01-10), pages 450 - 459, XP055099533, ISSN: 0969-7128, DOI: 10.1038/gt.2012.101
- MATTHEW RJ MASON ET AL: "Comparison of AAV Serotypes for Gene Delivery to Dorsal Root Ganglion Neurons", MOLECULAR THERAPY, APRIL 2010, vol. 18, no. 4, 23 February 2010 (2010-02-23), pages 715 - 724, XP055099555, ISSN: 1525-0016, DOI: 10.1038/mt.2010.19
- B. STOREK ET AL: "Sensory neuron targeting by self-complementary AAV8 via lumbar puncture for chronic pain", PROCEEDINGS OF THE NATIONAL ACADEMY OF SCIENCES, vol. 105, no. 3, 22 January 2008 (2008-01-22), pages 1055 - 1060, XP055099588, ISSN: 0027-8424, DOI: 10.1073/pnas.0708003105
- BLITS B ET AL: "Adeno-associated viral vector (AAV)-mediated gene transfer in the red nucleus of the adult rat brain: Comparative analysis of the transduction properties of seven AAV serotypes and lentiviral vectors", JOURNAL OF NEUROSCIENCE METHODS, ELSEVIER SCIENCE PUBLISHER B.V., AMSTERDAM, NL, vol. 185, no. 2, 15 January 2010 (2010-01-15), pages 257 - 263, XP026819079, ISSN: 0165-0270, [retrieved on 20091020]
- DARSHONG LIN ET AL: "Central Nervous System-directed AAV2/5-Mediated Gene Therapy Synergizes with Bone Marrow Transplantation in the Murine Model of Globoid-cell Leukodystrophy", MOLECULAR THERAPY, vol. 15, no. 1, January 2007 (2007-01-01), pages 44 - 52, XP055099624, ISSN: 1525-0016, DOI: 10.1038/sj.mt.6300026
- STOREK BENJAMIN ET AL: "Intrathecal long-term gene expression by self-complementary adeno-associated virus type 1 suitable for chronic pain studies in rats", MOLECULAR PAIN, BIOMED CENTRAL, LONDON, GB, vol. 2, no. 1, 30 January 2006 (2006-01-30), pages 4, XP021019474, ISSN: 1744-8069, DOI: 10.1186/1744-8069-2-4
- FEDERICI T ET AL: "Robust spinal motor neuron transduction following intrathecal delivery of AAV9 in pigs", GENE THERAPY, 2012 NATURE PUBLISHING GROUP, GB, vol. 19, 15 September 2011 (2011-09-15), pages 852 - 859, XP002711717, ISSN: 0969-7128, [retrieved on 20110915], DOI: 10.1038/GT.2011.130
- ABDELWAHED CHTARTO ET AL: "A next step in adeno-associated virus-mediated gene therapy for neurological diseases: regulation and targeting", BRITISH JOURNAL OF CLINICAL PHARMACOLOGY, JULY 2012, vol. 76, no. 2, 18 January 2013 (2013-01-18), pages 217 - 232, XP055165696, ISSN: 0306-5251, DOI: 10.1111/bcp.12065
- DAR-SHONG LIN ET AL: "CNS-targeted AAV5 gene transfer results in global dispersal of vector and prevention of morphological and function deterioration in CNS of globoid cell leukodystrophy mouse model", MOLECULAR GENETICS AND METABOLISM, ACADEMIC PRESS, AMSTERDAM, NL, vol. 103, no. 4, 6 May 2011 (2011-05-06), pages 367 - 377, XP028249738, ISSN: 1096-7192, [retrieved on 20110512], DOI: 10.1016/J.YMGME.2011.05.005

## Description

### Field of the invention

The present invention relates to the fields of virology and gene therapy. In particular, the invention relates to an adeno-associated virus gene therapy vector for use as a medicament for treating CNS disorders in a human, wherein the gene therapy vector comprises AAV serotype 5 capsid proteins and a gene product of interest flanked by AAV inverted terminal repeats (ITRs) and wherein the gene product of interest is operably linked to expression control elements comprising a promoter that produces sufficient expression of the gene product of interest to obtain a therapeutic effect, and wherein the gene therapy vector is administered by intrathecal lumbar administration, wherein 1 × 10¹⁴ - 1 × 10¹⁶ genome copies per kilogram of body weight is administered to the subject.

### Background of the invention

The central nervous system (CNS) is a complex system including the brain and spinal cord of a vertebrate. The peripheral nervous system is the part of the nervous system outside the brain and spinal cord. The spinal cord conducts sensory information from the peripheral nervous system to the brain and conducts motor information from the brain to various effectors.

Gene therapy using viral vectors has been investigated for delivery of therapeutic agents for treatment of various disorders concerning the CNS. Adeno-associated virus (AAV) vectors have a number of advantages, including low toxicity and immunogenicity and long-term expression of transgenes in the CNS (Kaplitt et al (1994) Nat Genet 8:148-154; Bartlett et al. (1998) Hum Gene Ther 9:1181-1186; Passini et al.(2002) J Neurosci 22:6437-6446). Therefore, AAV are further investigated for CNS gene therapy. Fagoe et al. (Gene Therapy (2014) 21, 242-252) report a dual promoter adeno-associated viral vector for delivery of two genes to dorsal root ganglion neurons. Reddy et al. (DOI: 10.1523/JNEUROSCI.1802-11.2011) describe bone marrow transplantation which augments the effect of brain- and spinal cord-directed adeno-associated virus 2/5 gene therapy by altering inflammation in the murine model of globoid-cell leukodystrophy. Lin et al. (DOI: 10.1016/J.YMTHE.2005.04.019) describe how an AAV2/5 vector expressing galactocerebrosidase ameliorates CNS disease. Watson et al. (DOI: 10.1038/sj.gt.3302735) describe intrathecal administration of AAV vectors for the treatment of lysosomal storage in the brains of MPSI mice. Gray (DOI: 10.1016/j.neuropharm.2012.06.024) describes gene therapy and neurodevelopmental disorders. Tenenbaum et al. (DOI: 10.1002/JGM.506) describe recombinant AAV-mediated gene delivery to the CNS. Mason et al. (DOI: 10.1038/mt.2010.19) describe a comparison of AAV serotypes for gene delivery. US 2004/258671 describes methods of treating pain by delivery of anti-inflammatory cytokines etc., which agents can be delivered using gene therapy techniques.

Many CNS diseases, for example amyotrophic lateral sclerosis (ALS, also known as motor neurone disease (MND)), affect both the cortical and the spinal motor neurons that are distributed over a wide area in the CNS. It has often been seen that viral vectors injected into the CNS transduce cells in the vicinity of the injection site, but not in the wide area that is required. Furthermore, injection into the brain is a surgically invasive procedure subject to safety concerns. Some studies are performed to assess gene therapy using lumbar punction. Drawbacks reported in the art are that gene transfer by lumbar punction failed to transduce neuronal tissue but only transduced meningeal fibroblasts unless injected intraparenchymally (Finegold (1999) Hum Gene Ther 10:1251-1257; Milligan (2005) Eur J Neurosci 21:2136-2148; Milligan (2005) Mol Pain 1:9; Milligan (2006) Pain 126:294-308), resulted in transgene expression for less than 2 weeks (Milligan (2005) Eur J Neurosci 21:2136-2148; Milligan (2005) Mol Pain 1:9; Lin (2002) Neurosci Lett 317:1-4; Lin (2002) Gene Ther 9:1247-1253), requires multiple injections (Milligan (2006) Pain 126:294-308) or requires a pretreatment (Vulchanova (2010) Molecular Pain 6:31).

Storek and coworkers (Storek (2008) PNAS 105(3):1055-1060) disclose that they were unable to detect transgene expression with conventional single-stranded rAAV2 and that therefore they have tested a variety of rAAV vector modifications: pseudotyping with capsids of different serotypes and the double-stranded, self-complementary rAAV. Specifically, Storek et al disclose that self-complementary AAV8 efficiently and selectively transduced the primary sensory neurons in the dorsal root ganglia, but not the brain, upon intrathecal administration and established long-term gene expression after a single vector administration.

Thus, there is still a need in the art for methods of gene transfer that can be used in the treatment or prophylaxis of neurological diseases in mammals. In particular, there is a need for methods of gene transfer to transduce neuronal tissue that does not suffer from one or more of the drawbacks indicated above.

### Description of the invention

### Brief description of the invention

The invention is set out in the appended set of claims.

### Definitions

A "nucleic acid construct" is defined as a nucleic acid molecule which is isolated from a naturally occurring gene or which has been modified to contain segments of nucleic acids which are combined or juxtaposed in a manner which would not otherwise exist in nature. A nucleic acid molecule is represented by a nucleotide sequence. Optionally, a nucleotide sequence present in a nucleic acid construct is operably linked to one or more control sequences, which direct the production or expression of said peptide or polypeptide in a cell or in a subject.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species. The term "heterologous" may be used to indicate that in nature the nucleic acid or polypeptide molecule is produced by a host cell or organisms of a different species.

"Expression control sequence" refers to a nucleic acid sequence that regulates the expression of a nucleotide sequence to which it is operably linked. An expression control sequence is "operably linked" to a nucleotide sequence when the expression control sequence controls and regulates the transcription and/or the translation of the nucleotide sequence. Thus, an expression control sequence can include promoters, enhancers, internal ribosome entry sites (IRES), transcription terminators, a start codon in front of a protein-encoding gene, splicing signals for introns, and stop codons. The term "expression control sequence" is intended to include, at a minimum, a sequence whose presence are designed to influence expression, and can also include additional advantageous components. For example, leader sequences and fusion partner sequences are expression control sequences. The term can also include the design of the nucleic acid sequence such that undesirable, potential initiation codons in and out of frame, are removed from the sequence. It can also include the design of the nucleic acid sequence such that undesirable potential splice sites are removed. It includes sequences or polyadenylation sequences (pA) which direct the addition of a polyA tail, i.e., a string of adenine residues at the 3'-end of a mRNA, which may be referred to as polyA sequences. It also can be designed to enhance mRNA stability. Expression control sequences which affect the transcription and translation stability, e.g., promoters, as well as sequences which effect the translation, e.g., Kozak sequences, suitable for use in insect cells are well known to those skilled in the art. Expression control sequences can be of such nature as to modulate the nucleotide sequence to which it is operably linked such that lower expression levels or higher expression levels are achieved.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter, including e.g. attenuators or enhancers, but also silencers. A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is only active in specific types of tissues or cells.

A "3' UTR" or "3' non-translated sequence" (also often referred to as 3' untranslated region, or 3'end) refers to the nucleic acid sequence found downstream of the coding sequence of a gene, which comprises, for example, a transcription termination site and (in most, but not all eukaryotic mRNAs) a polyadenylation signal (such as e.g. AAUAAA or variants thereof). After termination of transcription, the mRNA transcript may be cleaved downstream of the polyadenylation signal and a poly(A) tail may be added, which is involved in the transport of the mRNA to the cytoplasm (where translation takes place).

A "vector" is a nucleic acid molecule (typically DNA or RNA) that serves to transfer a passenger nucleic acid sequence (i.e., DNA or RNA) into a host cell. Three common types of vectors include plasmids, phages and viruses. Preferably, the vector is a virus. Vectors that contain both a promoter and a cloning site into which a polynucleotide can be operatively linked are well known in the art. Such vectors are capable of transcribing RNA *in vitro* or *in vivo*, and are commercially available from sources such as Stratagene (La Jolla, Calif.) and Promega Biotech (Madison, Wis.). In order to optimize expression and/or in vitro transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

A "viral vector" refers to a vector comprising some or all of the following: viral genes encoding a gene product, control sequences and viral packaging sequences. A "parvoviral vector" is defined as a recombinantly produced parvovirus or parvoviral particle that comprises a polynucleotide to be delivered into a host cell, either *in vivo*, *ex vivo* or *in vitro.* Examples of parvoviral vectors include e.g., adeno-associated virus vectors. Herein, a parvoviral vector construct refers to the polynucleotide comprising the viral genome or part thereof, and a transgene.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is only active in specific types of tissues or cells.

The promoter may be any appropriate promoter sequence, which shows transcriptional activity in the cell, including mutant, truncated, and hybrid promoters, and may be obtained from genes encoding extra-cellular or intracellular polypeptides either homologous (native) or heterologous (foreign) to the cell.

The promoter may be the promoter natively associated with the coding sequence to be expressed. The promoter may also be a constitutive or inducible promoter foreign to the coding sequence to be expressed. Examples of suitable promoters for use in mammalian cells are e.g. described in Sambrook and Russell (2001) "Molecular Cloning: A Laboratory Manual (3rd edition), Cold Spring Harbor Laboratory, Cold Spring Harbor Laboratory Press, New York. Examples of suitable promoters for use in yeasts include e.g. glycolytic promoters.

The term "flanked" with respect to a sequence that is flanked by another element(s) herein indicates the presence of one or more of the flanking elements upstream and/or downstream, i.e., 5' and/or 3', relative to the sequence. The term "flanked" is not intended to indicate that the sequences are necessarily contiguous. For example, there may be intervening sequences between the nucleic acid encoding the transgene and a flanking element. A sequence that is "flanked" by two other elements (e.g. ITRs), indicates that one element is located 5' to the sequence and the other is located 3' to the sequence; however, there may be intervening sequences therebetween. In a preferred embodiment a nucleotide sequence of (i) is flanked on either side by parvoviral inverted terminal repeat nucleotide sequences.

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or isochemically modified, non-natural, or derivatized nucleotide bases. "Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as oligomers or oligos and may be isolated from genes, or chemically synthesized by methods known in the art.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment," as used herein, covers any treatment of a disease in a mammal, particularly in a human, and incudes:
(a) preventing the disease from occurring in a subject which may be predisposed to the disease but has not yet been diagnosed as having it;
(b) inhibiting the disease, i.e., arresting its development; and
(c) relieving the disease, i.e., causing regression of the disease.

"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as those using the Smith Waterman algorithm, are preferred. Alternatively percentage similarity or identity may be determined by searching against public databases, using algorithms such as FASTA, BLAST, etc.

### Detailed description of the invention

The delivery of a gene to the CNS has so far been hampered by the size and complexity of the CNS. Following a single injection into the brain parenchyma, expression of the transgene is local and remains mainly restricted to the infused area. Using the CSF as a method for delivery of the viral vector, a larger area can be reached. The current study shows that using an AAV-5 vector, it is possible to reach this goal of transduction over a larger area in the CNS. The treatment was well tolerated as no adverse clinical signs and no obvious neuronal loss were observed. Both neuronal and glial cells were transduced using a ubiquitous CAG promoter and no transgene expression was observed outside the CNS. Using cell specific promoters, such as the GFAP promoter or synapsin-1 promoter, one can direct the expression to a specific population. We have shown that areas of the cortex, cerebellum and subventricular zone show expression of the transgene in both neurons and glial cells. In the spinal cord, motorneuron transduction was prevalent as well as transduction of neurons in the dorsal root ganglia. These results show that the method of CNS-mediated delivery can be used to deliver genes for a gene therapeutic approach. Indications that could be helped by this could be: motorneuron diseases, sensory related indications and a variety of other neurological indications.

In a first aspect, the present invention relates to an adeno-associated virus (AAV) gene therapy vector for use as a medicament in a mammalian subject, wherein the mammalian subject is human, wherein the gene therapy vector comprises AAV serotype 5 capsid proteins and a gene product of interest flanked by AAV inverted terminal repeats (ITRs) and wherein the gene product of interest is operably linked to expression control elements comprising a promoter that produces sufficient expression of the gene product of interest to obtain a therapeutic effect, and wherein the gene therapy vector is administered by intrathecal lumbar administration, wherein 1 × 10¹⁴ - 1 × 10¹⁶ genome copies per kilogram of body weight is administered to the subject. The invention further pertains to an AAV gene therapy vector as herein defined above, for use in the treatment of a disorder, such as for example a motorneuron disease, a sensory related indication and a variety of other neurological disorders.

Viruses of the Parvoviridae family are small DNA animal viruses. The family Parvoviridae may be divided between two subfamilies: the Parvovirinae, which infect vertebrates, and the Densovirinae, which infect insects. Members of the subfamily Parvovirinae are herein referred to as the parvoviruses and include the genus Dependovirus. As may be deduced from the name of their genus, members of the Dependovirus are unique in that they usually require co-infection with a helper virus such as adenovirus or herpes virus for productive infection in cell culture. The genus Dependovirus includes AAV, which normally infects humans (e.g., serotypes 1, 2, 3A, 3B, 4, 5, and 6) or primates (e.g., serotypes 1 and 4), and related viruses that infect other warm-blooded animals (e.g., bovine, canine, equine, and ovine adeno-associated viruses). Further information on parvoviruses and other members of the Parvoviridae is described in Kenneth I. Berns, "Parvoviridae: The Viruses and Their Replication," Chapter 69 in Fields Virology (3d Ed. 1996).

The genomic organization of all known AAV serotypes is very similar. The genome of AAV is a linear, single-stranded DNA molecule that is less than about 5,000 nucleotides (nt) in length. Inverted terminal repeats (ITRs) flank the unique coding nucleotide sequences for the non-structural replication (Rep) proteins and the structural (VP) proteins. The VP proteins (VP1, -2 and -3) form the capsid. The terminal 145 nt are self-complementary and are organized so that an energetically stable intramolecular duplex forming a T-shaped hairpin may be formed. These hairpin structures function as an origin for viral DNA replication, serving as primers for the cellular DNA polymerase complex. Following wild-type (wt) AAV infection in mammalian cells the Rep genes (i.e. Rep78 and Rep52) are expressed from the P5 promoter and the P19 promoter, respectively and both Rep proteins have a function in the replication of the viral genome. A splicing event in the Rep ORF results in the expression of actually four Rep proteins (i.e. Rep78, Rep68, Rep52 and Rep40). However, it has been shown that the unspliced mRNA, encoding Rep78 and Rep52 proteins, in mammalian cells are sufficient for AAV vector production. Also in insect cells the Rep78 and Rep52 proteins suffice for AAV vector production.

A "recombinant parvoviral or AAV vector" (or "rAAV vector") herein refers to a vector comprising one or more polynucleotide sequences of interest, a gene product of interest, genes of interest or "transgenes" that are flanked by at least one parvoviral or AAV inverted terminal repeat sequences (ITRs). Such rAAV vectors can be replicated and packaged into infectious viral particles when present in an insect host cell that is expressing AAV rep and cap gene products (i.e. AAV Rep and Cap proteins). When an rAAV vector is incorporated into a larger nucleic acid construct (e.g. in a chromosome or in another vector such as a plasmid or baculovirus used for cloning or transfection), then the rAAV vector is typically referred to as a "pro-vector" which can be "rescued" by replication and encapsidation in the presence of AAV packaging functions and necessary helper functions. A gene product of interest is flanked by AAV ITRs on either side. Any AAV ITR may be used in the constructs of the invention, including ITRs from AAV1, AAV2, AAV4, AAV5, AAV6, AAV8 and/or AAV9. ITRs of AAV2 are most preferred. Examples of preferred ITR sequences for use in preferred nucleic acid constructs of the invention are given in SEQ ID NO: 1 (left or upstream ITR) and SEQ ID NO: 2 (right or downstream ITR).

AAV is able to infect a number of mammalian cells. See, e.g., Tratschin et al. (1985, Mol. Cell Biol. 5:3251-3260) and Grimm et al. (1999, Hum. Gene Ther. 10:2445-2450). However, AAV transduction of human synovial fibroblasts is significantly more efficient than in similar murine cells, Jennings et al., Arthritis Res, 3:1 (2001), and the cellular tropicity of AAV differs among serotypes. See, e.g., Davidson et al. (2000, Proc. Natl. Acad. Sci. USA, 97:3428-3432), who discuss differences among AAV2, AAV4, and AAV5 with respect to mammalian CNS cell tropism and transduction efficiency and see Goncalves, 2005, Virol J. 2(1):43 who discusses approaches to modification of AAV tropism.

An AAV gene therapy vector for use in the present invention may be produced either in mammalian cells or in insect cells. Both methods are described in the art. For example Grimm et al. (2003 Molecular Therapy 7(6):839-850) disclose a strategy to produce AAV vectors in a helper virus free and optically controllable manner, which is based on transfection of only two plasmids into 293T cells. They disclose a method for production of a hybrid AAV vector comprising AAV2 ITRs and AAV5 capsid proteins. Further information can also be found in Blits et al. (2010) (Journal of Neuroscience methods 185(2):257-263). The terms "hybrid" and "pseudotyped" are used interchangeably herein and are used to indicate vectors of which the Rep proteins, ITRs and/or capsid proteins are of different serotypes. For example, the ITRs and the Rep proteins are of AAV2 and the capsid proteins are of AAV5.The term "chimeric" is used herein to describe that a single gene, such as for example the capsid, is composed of at least two sequences derived from different serotypes.

AAV5 can for example be produced in mammalian cells according to the following method, but is not limited thereto: The vector genome contains the transgene expression cassette flanked by two inverted terminal repeats (ITRs) derived from AAV serotype 2. The total length of the viral vector genome may not exceed the wild type genome size of 4.7 kB in order to maintain efficient packaging efficiency. A single capsid is composed of 60 viral proteins of either, VP1 (62kDa), VP2 (73 kDa), or VP3 (87 kDa), at a ratio of 1:1:10. The manufacturing process of AAV vectors is based upon Ca(PO4)2 transfection of two plasmids into human embryonic kidney production cells (HEK293) in roller bottles (850 cm² surface area) followed by purification of the encapsidated vector genomes by filtration and chromatography techniques. The first plasmid is the viral vector plasmid and contains an expression construct which is flanked by AAV2 ITRs. The second plasmid is the packaging plasmid and encodes the AAV rep type 2 and cap type 5 genes of the desired serotype and adenovirus early helper genes E2A, VA, E4 (pDP5; nucleotide sequence disclosed in SEQ ID NO:3). The genome of the production cell line comprises the adenovirus E1 to provide helper functions. Following co-transfection with the two plasmids in Iscove's Modified Dulbecco's Medium (IMDM) containing 10% fetal calf serum (FCS), the cells are incubated for three days in serum-free Dulbecco's modified Eagle's medium (DMEM) to allow vector production to occur. Vector production in roller bottles on average results in yields of 3×10³ vector genomes per cell or 4×10¹¹ vector genomes per roller bottle (quantified by qPCR). Subsequently, the cell culture is lysed by a buffer containing Triton-X-100 and cell debris removed by low speed centrifugation. The clarified bulk is purified by AVB Sepharose affinity chromatography and formulated into PBS/5% Sucrose by concentration and diafiltration using a 400 kDa hollow fiber module (for example from Spectrum Laboratories).

Alternatively, an AAV gene therapy vector for use in the present invention may be produced in insect cells, as has been described previously by Urabe et al. (Journal of Virology 2006 80(4):1874-1885). Also modifications in the Rep and VP1, VP2 and VP3 sequences previously disclosed can be employed in the present invention, such as for example disclosed in international publications WO 2007/046703, WO 2007/148971, WO 2009/014445, WO 2009/104964 and/or WO 2011/112089.

AAV ITR and Rep sequences that may be used in the present invention for the production of rAAV vectors in insect cells can be derived from the genome of any AAV serotype. Generally, the AAV serotypes have genomic sequences of significant homology at the amino acid and the nucleic acid levels. This provides an identical set of genetic functions to produce virions which are essentially physically and functionally equivalent. For the genomic sequence of the various AAV serotypes and an overview of the genomic similarities see e.g. GenBank Accession number U89790; GenBank Accession number J01901; GenBank Accession number AF043303; GenBank Accession number AF085716; Chiorini et al. (1997, J. Vir. 71: 6823-33); Srivastava et al. (1983, J. Vir. 45:555-64); Chiorini et al. (1999, J. Vir. 73:1309-1319); Rutledge et al. (1998, J. Vir. 72:309-319); and Wu et al. (2000, J. Vir. 74: 8635-47). rAAV serotypes 1, 2, 3, 4 and 5 are preferred source of AAV nucleotide sequences for use in the context of the present invention. Preferably the AAV ITR sequences for use in the context of the present invention are derived from AAV1, AAV2, and/or AAV5. More preferably, the ITR sequences for use in the present invention are AAV2 ITR. Likewise, the Rep (Rep78/68 and Rep52/40) coding sequences are preferably derived from AAV1, AAV2, and/or AAV5, more preferably AAV2

AAV Rep and ITR sequences are particularly conserved among most serotypes. The Rep78 proteins of various AAV serotypes are e.g. more than 89% identical and the total nucleotide sequence identity at the genome level between AAV2, AAV3A, AAV3B, and AAV6 is around 82% (Bantel-Schaal et al., 1999, J. Virol., 73(2):939-947). Moreover, the Rep sequences and ITRs of many AAV serotypes are known to efficiently cross-complement (i.e., functionally substitute) corresponding sequences from other serotypes in production of AAV particles in mammalian cells. US2003148506 reports that AAV Rep and ITR sequences also efficiently cross-complement other AAV Rep and ITR sequences in insect cells.

The AAV VP proteins are known to determine the cellular tropicity of the AAV virion. The VP protein-encoding sequences are significantly less conserved than Rep proteins and genes among different AAV serotypes. The sequences coding for the viral proteins (VP) VP1, VP2, and VP3 capsid proteins for use in the context of the present invention are derived from AAV5. Most preferably, VP1, VP2 and VP3 are AAV5 VP1, VP2 and VP3. Alternatively, VP1, VP2 and VP3 are wild-type AAV5 sequences, such as shown for example in SEQ ID NO: 3 (nucleotide sequence) and SEQ ID NO:4 (amino acid sequence). The ability of Rep and ITR sequences to cross-complement corresponding sequences of other serotypes allows for the production of pseudotyped rAAV particles comprising the capsid proteins of one serotype (e.g., AAV5) and the ITR sequences of another AAV serotype (e.g., AAV2). Such pseudotyped rAAV particles are a part of the present invention. Herein, a pseudotyped rAAV particle may be referred to as being of the type "x/y", where "x" indicates the source of ITRs and "y" indicates the serotype of capsid, for example a 2/5 rAAV particle has ITRs from AAV2 and a capsid from AAV5.

Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having from about 75% to about 99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.

Although similar to other AAV serotypes in many respects, AAV5 differs from other human and simian AAV serotypes more than other known human and simian serotypes. In view thereof, the production of rAAV5 can differ from production of other serotypes in insect cells. Where methods are employed to produce rAAV5, it is preferred that one or more constructs comprise, collectively in the case of more than one construct, a nucleotide sequence comprising an AAV5 ITR, a nucleotide sequence comprises an AAV5 Rep coding sequence (i.e. a nucleotide sequence comprises an AAV5 Rep78) and/or an AAV5 Cap coding sequence. Such ITR, Rep and Cap sequences can be modified as desired to obtain efficient production of rAAV5 or pseudotyped rAAV5 vectors in insect cells. E.g., the start codon of the Rep sequences can be modified, VP splice sites can be modified or eliminated, and/or the VP1 start codon can be modified to improve the production of rAAV5 vectors in the insect cell, as is for example disclosed in WO 2007/046703, WO 2007/148971 and/or, WO 2009/014445. Also included in the present invention are chimeric AAV5 capsids, wherein for example VP1 of AAV5 is partially or fully replaced by VP1 derived of AAV2 and VP2 and 3 are derived of AAV5 (Urabe et al., 2006; WO2000/028004). In such a chimeric AAV5 capsid, at least VP3 should be AAV5 or derived from AAV5, whereas one or both of VP1 and VP2 may be a different AAV serotype or derived from a different AAV serotype.

Preferred adenoviral vectors are modified to reduce the host response as reviewed by Russell (2000, J. Gen. Virol. 81: 2573-2604), or as described in US20080008690 and by Zaldumbide and Hoeben (Gene Therapy 2008:239-246).

Preferably a Rep78 protein having the nucleic acid sequence of SEQ ID NO: 5 and/or an amino acid sequence according to SEQ ID NO: 6 is employed in the present invention and a Rep52 protein having the nucleic acid sequence of SEQ ID NO: 7 is employed in the present invention.

"Intrathecal" administration is used to refer to intrathecal injection, i.e. a dministration within the cerebrospinal fluid at any level of the cerebrospinal axis, including injection into the cerebral ventricles (see also "Route of Administration". Data Standards Manual. Food and Drug Administration. Retrieved 11 March 2011). This is an injection through the theca of the spinal cord into the subarachnoid space so that it reaches the CSF. This method of administration is useful in for example spinal anaesthesia, chemotherapy, or pain management applications. This route is also used to introduce drugs that fight certain infections, particularly post-neurosurgical. A substance administered via intrathecal injection avoids the need to passage the blood brain barrier. Drugs given intrathecally often do not contain any preservative or other potentially harmful inactive ingredients that are sometimes found in medicaments for intravenous injection. The intrathecal method of delivery is regarded as less invasive than infusion into the central nervous system (CNS) tissue itself, because infusion into the CNS requires complex brain surgery. Intrathecal delivery can be performed without the need of a specialized center for brain injections.

The neck region of the spine is known as the *Cervical Spine.* This region consists of seven vertebrae, which are abbreviated C1 through C7 (top to bottom). These vertebrae protect the brain stem and the spinal cord, support the skull, and allow for a wide range of head movement. The first cervical vertebra (C1) is called the Atlas. The Atlas is ring-shaped and it supports the skull. C2 is called the Axis. It is circular in shape with a blunt tooth-like structure (called the *Odontoid Process* or dens) that projects upward into the Atlas. Together, the Atlas and Axis enable the head to rotate and turn. The other cervical vertebrae (C3 through C7) are shaped like boxes with small spinous processes (finger-like projections) that extend from the back of the vertebrae. Beneath the last cervical vertebra are the 12 vertebrae of the *Thoracic Spine.* These are abbreviated T1 through T12 (top to bottom). T1 is the smallest and T12 is the largest thoracic vertebra. The thoracic vertebrae are larger than the cervical bones and have longer spinous processes. In addition to longer spinous processes, rib attachments add to the thoracic spine's strength. These structures make the thoracic spine more stable than the cervical or lumbar regions. In addition, the rib cage and ligament system limit the thoracic spine's range of motion and protects many vital organs. The *Lumbar Spine* has 5 vertebrae abbreviated L1 through L5 (largest). The size and shape of each lumbar vertebra is designed to carry most of the body's weight. Each structural element of a lumbar vertebra is bigger, wider and broader than similar components in the cervical and thoracic regions. The lumbar spine has more range of motion than the thoracic spine, but less than the cervical spine. The lumbar facet joints allow for significant flexion and extension movement but limits rotation. The *Sacrum* is located behind the pelvis. Five bones, abbreviated S1 through S5, fused into a triangular shape, form the sacrum. The sacrum fits between the two hip bones connecting the spine to the pelvis. The last lumbar vertebra (L5) articulates (moves) with the sacrum. Immediately below the sacrum are five additional bones, fused together to form the *Coccyx* (tailbone).

In the present invention, the gene therapy vector is administered by intrathecal lumbar administration. More preferably, the intrathecal lumbar administration is at a position selected from the group consisting of: L4 - L5, L3 - L 4, L1 - L2 and L2 - L3. Most preferably, the gene therapy vector is administered by intrathecal injection between L4 and L5. In one preferred embodiment, the gene therapy vector is administrated only by lumbar administration, i.e. there is no administration at other locations.

In one preferred embodiment, the gene therapy vector is for administration into the *cisterna magna* which is performed in combination with the intrathecal administration, or in other words prior to, simultaneous or after the intrathecal administration.

In another preferred embodiment, administration into the *cisterna magna* is performed in combination with the lumbar administration, whereby preferably administration into the *cisterna magna* is performed prior to, simultaneous or after the lumbar administration. Preferably in this embodiment, the lumbar administration is in the intrathecal region.

In a most preferred embodiment, the gene therapy vector is administered only into the *cisterna magna* in combination with the lumbar administration, i.e. there is no administration at other locations than *cisterna magna* and lumbar. Preferably in this embodiment, the lumbar administration is in the intrathecal region.

The "cisterna magna" or "cerebellomedullary cistern" is one of three principal openings in the subarachnoid space between the arachnoid and pia mater layers of the meninges surrounding the brain. The openings are collectively referred to as cisterns. The cisterna magna is located between the cerebellum and the dorsal surface of the medulla oblongata. Cerebrospinal fluid produced in the fourth ventricle drains into the cisterna magna via the lateral apertures and median aperture.

Combined administration, or administration "prior to, simultaneous or after" intrathecal administration as used herein, means that administration into the cisterna magna and intrathecal administration take place within a time span of preferably less than 2 weeks, 1 week, 4 days, 48 hours, 24 hours, 12 hours, 6 hours, 4 hours, 2 hours, 1 hour, 30 minutes, 20 minutes, 10 minutes or 5 minutes.

Recombinant AAV vectors can either have single-stranded DNA or double-stranded DNA. Single-stranded DNA (ssDNA) AAV genome must be converted into into double-stranded DNA (dsDNA) prior to expression of the encoded transgene. This step can be circumvented by the use of self-complementary vectors or monomeric duplex vectors which package an inverted repeat vector genome that folds into dsDNA without requiring DNA synthesis or base- pairing between multiple vector genomes, thereby increasing efficiency of AAV- mediated gene transfer. For a review of self-complementary AAV vectors, see e.g., McCarty, D.M. Molec. Ther. (2008) 16:1648-1656. The so-called self-complementary AAV vectors (WO2001/092551) contain at least one modified ITR, preferably modified by deletion of the terminal resolution site (trs) and differ conceptionally from monomeric duplex AAV vectors which are enabled to be produced using only intact AAV ITR's (WO2011/122950). In a preferred embodiment, the self-complementary AAV vectors do not fall within the scope of the present invention. In contrast, a monomeric duplex vector, as said above is different in sequence from self-complementary vector, and can be advantageously employed in the present invention as it concerns vector genomes that besides the intact ITR sequences are half (1/2) the size or less than the wild type size of AAV vectors (ie 4.8 kb). This has the advantage that multiple repeats (concatemers of 2, 4, 8 or more times the expression unit) can be built in the AAV vector resulting in mono, -duplo, - quadromeric duplex vectors. An example of expression units in such vectors could be vectors containing shRNA's or miRNA's. In the present invention it has been shown that broad transduction of brain tissue can be obtained after lumbar injection, using a single-stranded AAV gene therapy vector. Therefore, it is a preferred embodiment of the invention that the AAV gene therapy vector is a single stranded AAV gene therapy vector.

The AAV gene therapy vector of the present invention achieves transduction over a large area in the central nervous system after intrathecal lumbar administration. Using an ubiquitous promoter (CAG promoter), both neuronal and glial cells were transduced in the cortex, cerebellum and subventricular zone. In addition, in the spinal cord, motorneuron transduction was prevalent as well as transduction of neurons in the dorsal root ganglia. Hence, the method of treatment enabled by the present invention can be used in the treatment of several CNS disorders. In a preferred embodiment, an AAV gene therapy vector according to the present invention comprises a gene product for interest that can be used in the treatment or prophylaxis of a condition selected from the group consisting of: amyotrofic laterale sclerose (ALS), spinal muscular atrophy (SMA), pain, lysosomal storage diseases (LSD), Huntington's disease, Alzheimer's disease, Tay-Says disease, Friedreich ataxia, ataxia telangietacsia, Spinocerebellar ataxia type 1, 2 and 3, Niemann-Pick disease A, B and C, Dopa-responsive dystonia, Fragile X syndrome, Krabbe disease, Glycogen storage disease type 2 (Pompe), Primary lateral sclerosis, Pelizaeus-Merzbacher disease, X-linked adrenoleukodystrophy, Giant axonal neuropathy, Multiple system atrophy (MSA), Proximal myotonic myopathy, Neuronal Ceroid Lipofuscinosis (Batten disease) and various forms of CNS cancer, such as for example, primary CNS lymphoma, metastatic or secondary brain tumors, primary spinal cord tumors. CNS cancer include for example glioblastoma, astrocytoma, oligodendroglioma, ependymoma, meningioma, medulloblastoma, ganglioglioma, schwannoma, craniopharyngioma, chordoma, non-Hodgkin CNS lymphoma.

A group of metabolic disorders known as lysosomal storage diseases (LSD) includes over forty genetic disorders, many of which involve genetic defects in various lysosomal hydrolases. Representative lysosomal storage diseases and the associated defective enzymes are listed in Table 1.

**Table 1**

| ***Lysosomal storage disease*** | ***Defective enzyme*** |
|---|---|
| Aspartylglucosaminuria | Aspartylglucosaminidase |
| Fabry | α-Galactosidase A |
| Infantile Batten Disease* (CNL1) | Palmitoyl Protein Thioesterase |
| Classic Late Infantile Batten Disease* (CNL2) | Tripeptidyl Peptidase |
| Juvenile Batten Disease* (CNL3) | Lysosomal Transmembrane Protein |
| Batten, other forms* (CNL4-CNL8) | Multiple gene products |
| Cystinosis | Cysteine transporter |
| Farber | Acid ceramidase |
| Fucosidosis | Acid α-L-fucosidase |
| Galactosidosialidosis | Protective protein/cathepsin A |
| Gaucher types 1, 2*, and 3* | Acid β-glucosidase, or glucocerebrosidase |
| G_{M1} gangliosidosis* | Acid β-galactosidase |
| Hunter* | Iduronate-2-sulfatase |
| Hurler-Scheie* | α-L-Iduronidase |
| Krabbe* | Galactocerebrosidase |
| α-Mannosidosis* | Acid α-mannosidase |
| β-Mannosidosis* | Acid β-mannosidase |
| Maroteaux-Lamy | Arylsulfatase B |
| Metachromatic leukodystrophy* | Arylsulfatase A |
| Morquio A | N-Acetylgalactosamine-6-sulfate sulfatase |
| Morquio B | Acid β-galactosidase |
| Mucolipidosis II/III* | N-Acetylglucosamine-1-phosphotran sferase |
| Niemann-Pick A*, B | Acid sphingomyelinase |
| Niemann-Pick C* | NPC-1 |
| Pompe* | Acid α-glucosidase |
| Sandhoff* | β-Hexosaminidase B |
| Sanfilippo A* | Heparan N-sulfatase |
| Sanfilippo B* | α-N-Acetylglucosaminidase |
| Sanfilippo C* | Acetyl-CoA:α-glucosaminide N-acetyltransferase |
| Sanfilippo D* | N-Acetylglucosamine-6-sulfate sulfatase |
| Schindler Disease* | α-N-Acetylgalactosaminidase |
| Schindler-Kanzaki | α-N-Acetylgalactosaminidase |
| Sialidosis | α-Neuramidase |
| Sly* | β-Glucuronidase |
| Tay-Sachs* | β-Hexosaminidase A |
| Wolman* | Acid Lipase |
| * CNS involvement | |

In another preferred embodiment, the gene product of interest is for use in the treatment or prophylaxis of a condition associated with motor neurons and/or neurons in the DRGs. These diseases include, but are not limited to pain, Amyotrophic lateral sclerosis (ALS), Primary lateral sclerosis (PLS), Progressive muscular atrophy (PMA), Progressive bulbar palsy (PBP) and Pseudobulbar palsy.

In a particularly preferred embodiment, an AAV gene therapy vector according to the present invention comprises a gene product for interest that can be used in the treatment or prophylaxis of a condition selected from the group consisting of: amyotrofic laterale sclerose (ALS), spinal muscular atrophy (SMA), Huntington's disease, Multiple system atrophy (MSA) and Lysosomal storage diseases selected from the group consisting of: Fabry, Juvenile Batten Disease (CNL3), Gaucher types 1, 2, and 3, Hunter, Pompe, Sanfilippo A and Sanfilippo B.

Hence, in a preferred embodiment, the gene product of interest is selected from the group consisting of: Aspartylglucosaminidase, α-Galactosidase A, Palmitoyl Protein Thioesterase, Tripeptidyl Peptidase, Lysosomal Transmembrane Protein, Multiple gene products, Cysteine transporter, Acid ceramidase, Acid α-L-fucosidase, Protective protein/cathepsin A, Acid β-glucosidase, or glucocerebrosidase, Acid β-galactosidase, Iduronate-2-sulfatase, α-L-Iduronidase, Galactocerebrosidase, Acid α-mannosidase, Acid β-mannosidase, Arylsulfatase B, Arylsulfatase A, N-Acetylgalactosamine-6-sulfate sulfatase, Acid β-galactosidase, N-Acetylglucosamine-1-phosphotran sferase, Acid sphingomyelinase, NPC-1, Acid α-glucosidase, β-Hexosaminidase B, Heparan N-sulfatase, α-N-Acetylglucosaminidase, Acetyl-CoA:α-glucosaminide N-acetyltransferase, N-Acetylglucosamine-6-sulfate sulfatase, α-N-Acetylgalactosaminidase, α-N-Acetylgalactosaminidase, α-Neuramidase, β-Glucuronidase, β-Hexosaminidase A, Acid Lipase, neurotrophic factors such as Nerve Growth Factor (NGF), Neurotrophin-3 (NT-3), Neurotrophin-4/5 (NT-4/5), Brain derived neurotrophic factor (BDNF), Cerebral Dopamine Neurotrophic factor (CDNF), glial cell line-derived neurotrophic factor (GDNF), ciliary neurotrophic factor (CNTF) and growth factor Insuline-like growth factor (IGF-1). Moreover, miRNA sequences may be used as well to downregulate expression of certain genes, such as for example in the treatment of Huntington's disease.

Most of the diseases mentioned herein, except for Huntington's disease, can be treated inter alia by provision of a corrected version of the defective gene. Huntington's disease however requires the downregulation of a certain gene, i.e. the Huntingtin gene (also known as HTT, HD and IT15) located on chromosome 4 in humans. Subjects affected with Huntington 's disease carry a HTT gene with more than 36 trinucleotide CAG repeats at the 5' end of the gene. Approaches for the development of RNAi-based Huntington Disease therapy include total HTT knockdown (of both mutant and wild type gene) by for example targeting exon 1. Other approaches are for example allele specific silencing of only mutant HTT by targeting the CAG repeats in HTT exon 1 and allele-specific inhibition of mutant HTT expression by for example targeting a SNP in exon 67.

In a preferred embodiment the gene product of interest is operably linked to expression control elements comprising a promoter that produces sufficient expression of the gene product of interest to obtain a therapeutic effect. The level of transgene expression in eukaryotic cells is largely determined by the transcriptional promoter within the transgene expression cassette. Promoters that show long-term activity and are tissue and even cell-specific are used in some embodiments. Nonlimiting examples of promoters include, but are not limited to, the cytomegalovirus (CMV) promoter (Kaplitt et al. (1994) Nat. Genet., 8:148-164), CMV/human β3-globin promoter (Mandel et al. (1998) J. Neurosci., 18:4271-4284), phosphoglycerate kinase (PGK) promoter, CAG promoter (a combination of the cytomegalovirus early enhancer element and chicken beta-actin promoter) (Klein et al. (2008) Mol Ther 16(1):89-96; Shevtsova et al (2004) Exp Physiol 90:53-59), GFAP promoter (Xu et al. (2001) Gene Ther., 8:1323-1332; Lee et al. (2008) Glia 56:481-493), synapsin-1 promoter (Kuegler et al. (2001) Mol Cell Neurosci 17: 78-96; Drinkut et al. (2012) Molecular Therapy 20:534-543), the 1.8-kb neuron-specific enolase (NSE) promoter (Klein et al. (1998) Exp. Neurol., 150:183-194), chicken beta actin (CBA) promoter (Miyazaki (1989) Gene, 79:269-277) and the β-glucuronidase (GUSB) promoter (Shipley et al. (1991) Genetics, 10:1009-1018). To prolong expression, other regulatory elements may additionally be operably finked to the transgene, such as, e.g., the Woodchuck Hepatitis Virus Post-Regulabory Element (WPRE) (Donello et al. (1998) J. Virol., 72, 5085-5092) or the bovine growth hormone (BGH) polyadenylation site. Drinkut and coworkers applied a glial (GFAP) promoter to express GDNF and showed that that was more beneficial than expressing this from a neuron specific promoter (Drinkut et al., 2012, *supra*). Alternatively or in addition to use of a specific promoter, regulated expression could be achieved using regulatable expression elements such as for example the Gene Switch system (Maddalena et al.(2013) Molecular Therapy - Nucleic Acids 2:e106) or derivates of the the tetracyclin operon (Gossen and Bujard (1992) PNAS 89:5547-5551).

In a preferred embodiment of the present invention, the AAV gene therapy vector comprises AAV5 capsid proteins, AAV2 ITRs and a gene product of interest located between the ITRs. Preferably the gene therapy vector is administered by lumbar injection, possibly in combination with injection into the cisterna magna. Preferred gene products and diseases to be treated (either as treatment or as prophylaxis) using this preferred AAV gene therapy vector are selected from the group consisting of: GDNF or IGF-1 for the treatment of ALS, GDNF or IGF-1 for the treatment of SMA, miRNA for downregulation of the faulty Spinal motorneuron survival protein (SMN) gene for the treatment of SMA, miRNA to achieve allele-specific downregulation or downregulation of both alleles of HTT for the treatment of Huntington's disease, GDNF for the treatment of Huntington's disease, GDNF for the treatment of MSA, α-Galactosidase A for the treatment of Fabry, Lysosomal Transmembrane Protein for the treatment of Juvenile Batten Disease (CNL3), Acid β-glucosidase or glucocerebrosidase for the treatment of Gaucher types 1, 2, and 3, Iduronate-2-sulfatase for the treatment of Hunter, Acid α-glucosidase for the treatment of Pompe, Heparan N-sulfatase for the treatment of Sanfilippo A and α-N-Acetylglucosaminidase for the treatment of Sanfilippo B.

A therapeutically effective amount of an AAV gene therapy vector or pharmaceutical composition of the invention may be given to a patient in need thereof.

The AAV gene therapy vector of the invention will typically be included in a pharmaceutical composition, optionally in combination with a pharmaceutical carrier, diluent and/or adjuvant. Such compositions include the nucleic acid, nucleic acid construct, parvoviral virion or pharmaceutical composition in an effective amount, sufficient to provide a desired therapeutic or prophylactic effect, and a pharmaceutically acceptable carrier or excipient. An "effective amount" includes a therapeutically effective amount or a prophylactically effective amount.

A "therapeutically effective amount" refers to an amount effective, at dosages and for periods of time necessary, to achieve the desired therapeutic result, such as elevation of symptoms of the disorder. A therapeutically effective amount of a nucleic acid, nucleic acid construct, parvoviral virion or pharmaceutical composition may vary according to factors such as the disease state, age, sex, and weight of the individual, and the ability of the nucleic acid, nucleic acid construct, parvoviral virion or pharmaceutical composition to elicit a desired response in the individual. Dosage regimens may be adjusted to provide the optimum therapeutic response. A therapeutically effective amount is also typically one in which any toxic or detrimental effects of the nucleic acid, nucleic acid construct, parvoviral virion or pharmaceutical composition are outweighed by the therapeutically beneficial effects.

A "prophylactically effective amount" refers to an amount effective, at dosages and for periods of time necessary, to achieve the desired prophylactic result, such as preventing or inhibiting various conditions. A prophylactic dose may be used in subjects prior to or at an earlier stage of disease, and a prophylactically effective amount may be more or less than a therapeutically effective amount in some cases.

In particular embodiments, a range for therapeutically or prophylactically effective amounts of an AAV gene therapy vector or pharmaceutical composition may be 2 × 10¹⁴ gc/kg body weight of the subject. It is to be noted that dosage values may vary with the severity of the condition to be alleviated. For any particular subject, specific dosage regimens may be adjusted over time according to the individual need and the professional judgement of the person administering or supervising the administration of the compositions.

Preferably, the volume for infusion into the CSF in a human child is less than about 20cc and in a human adult less than about 30 cc. In non-human primates 6 cc can safely be delivered into a CSF total volume of 20 - 25 cc. Alternatively, at least half of the volume to be infused is aspirated and replaced with the gene therapy vector of the invention to reach a maximum of about 50 % volume replacement.

In addition, the AAV gene therapy vector of the invention can be administered in a concentrated form by standard methods known in the art, including but not limited to density-gradient centrifugation (eg CsCI, iodixanol), dialysis, ultracentrifugation, ion exchange (IEX) chromatography (eg anion, cation), gel filtration, affinity chromatography, Tangential Flow Filtration (TFF), Spin filter columns (eg Centricon). Also, adventitious virus or helper virus removal can be applied to improve the quality of the AAV gene therapy vector used in the present invention, such as for example described in WO 2013/036118.

As used herein "pharmaceutically acceptable carrier" or "exipient" includes any and all solvents, dispersion media, coatings, antibacterial and antifungal agents, isotonic and absorption delaying agents, and the like that are physiologically compatible. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound and/or the site of injection, use thereof in the pharmaceutical compositions of the invention is contemplated.

Supplementary active compounds can also be incorporated into the pharmaceutical compositions of the invention. Guidance on co-administration of additional therapeutics may for example be found in the Compendium of Pharmaceutical and Specialties (CPS) of the Canadian Pharmacists Association.

As e.g. indicated by Vulchanova (2010 Molecular Pain 6:31) viral vector administration by direct lumbar puncture has not been reported to result in appreciable transduction of spinal cord and DRG neurons. Vulchanova et al therefore suggest the pretreatment with mannitol to enhance the penetration of AAV5 and AAV8 vectors to the spinal cord parenchyma and to the cell bodies of DRG neurons. In particular, lumbar injection of AAV vector in mice at a dosage of about 4 × 10¹² vector genomes per kg requires intravenous pretreatment with mannitol prior to intrathecal lumbar administration of the vector in order to obtain expression of the reporter gene green fluorescent protein (GFP) in the spinal cord and select subsets of DRG neurons.

The pre-treatment with mannitol has as drawback that mannitol is known to cause side effects. In particular, adverse reactions more commonly reported during or after the infusion of mannitol include: Pulmonary congestion, fluid and electrolyte imbalance, acidosis, electrolyte loss, dryness of mouth, thirst, marked diuresis, urinary retention, edema, headache, blurred vision, convulsions, nausea, vomiting, rhinitis, arm pain, skin necrosis, thrombophlebitis, chills, dizziness, urticaria, dehydration, hypotension, tachycardia, fever and angina-like chest pains (Mannitol IV FDA Prescribing Information).

In a preferred embodiment of the present invention, the subject is not subjected to pretreatment prior to administration of the AAV gene therapy vector. In particular, the subject is not subjected to pretreatment with a substance that controls intracranial pressure and/or can disrupt the intercellular tight junctions between endothelial cells of the CNS microvasculature and/or facilitates delivery of drugs such as chemotherapeutics to the CNS. More preferably, the subject is not subjected to pretreatment with an osmotic agent prior to administration of the AAV gene therapy vectors. Osmotic agents include, but are not limited to urea, glycerol, sugars or sugar-alcohols such as polyols like mannitol and hypertonic saline. In particular, the subject is not subjected to pretreatment with mannitol, such as intravenous mannitol pretreatment prior to intrathecal lumbar administration of the AAV gene therapy vector.

In the present invention the mammalian subject is a human.

In an additional aspect, the disclosure relates to a method for producing a recombinant parvoviral (for example rAAV) virion (comprising a recombinant parvoviral (rAAV) vector as defined above) in an insect cell. Preferably, the method comprises the steps of: (a) culturing an insect cell as defined herein under conditions such that recombinant parvoviral (for example rAAV) vector is produced; and, (b) recovery of the recombinant parvoviral (for example rAAV) vector. It is understood here that the recombinant parvoviral (rAAV) vector produced in the method preferably is an infectious parvoviral or AAV virion that comprise the recombinant parvoviral (rAAV) vector nucleic acids. Growing conditions for insect cells in culture, and production of heterologous products in insect cells in culture are well-known in the art and described e.g. in the above cited references on molecular engineering of insects cells. Preferred methods and constructs for the production of rAAV virions of the invention are disclosed in e.g. WO2007/046703 and WO2007/148971.

Preferably the method for producing recombinant parvoviral virions further comprises the step of affinity-purification of the (virions comprising the) recombinant parvoviral (rAAV) vector using an anti-AAV antibody, preferably an immobilised antibody. The anti-AAV antibody preferably is an monoclonal antibody. A particularly suitable antibody is a single chain camelid antibody or a fragment thereof as e.g. obtainable from camels or llamas (see e.g. Muyldermans, 2001, Biotechnol. 74: 277-302). The antibody for affinity-purification of rAAV preferably is an antibody that specifically binds an epitope on an AAV capsid protein, whereby preferably the epitope is an epitope that is present on capsid proteins of more than one AAV serotype. E.g. the antibody may be raised or selected on the basis of specific binding to AAV2 capsid but at the same time also it may also specifically bind to AAV1, AAV3 and AAV5 capsids.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Description of the figures

Figure 1. schematic representation of the genome of the AAV vector. The expression cassette encoding for a GOI (gene of interest or gene product of interest), is under control of the CAG promoter, preceded by a Kozak sequence (gccaccatg...) and polyadenylated by a BGH polyadenylation signal. For the present examples, the GOI is green fluorescent protein (GFP). This cassette is flanked by two non-coding ITR sequences. Using recombinant baculoviruses for production, the expression cassete is packaged into an AAV-5 capsid.
Figure 2. Overview of transduction of the brain. Representative sections A-E are taken from the brain and stained for GFP. Expression is observed throughout the brain.
Figure 3. Higher magnification of the parietal cortex. Both neuronal and glial transduction can be observed several layers deep.
Figure 4. Higher magnification of the occipital cortex. Both neuronal and glial transduction can be observed several layers deep.
Figure 5. Higher magnification of the cerebellum. Many cells are positive for GFP.
Figure 6. Transduction of the cervical spinal cord. Sensory fibers terminating in the grey matter as well as ascending fibers as dots can be seen (a). Moreover, GFP positive motorneuron fibers (b) coming from the motorneuron pool can be observed (c). In (d) some glial cell are also stained positive for GFP.
Figure 7. Transduction of the thoracic spinal cord. Ascending sensory fibers fibers can be seen in the dorsal column (a). Moreover, GFP positive motorneuron fibers (b) coming from the motorneuron pool can be observed (c). In (d) some fibers are observed that can be both descending or ascending.
Figure 8. Transduction of the lumbar spinal cord. Sensory fibers fibers terimianting in the grey matter are visible (a). Moreover, GFP positive motorneurons in the motorneuronpool are present in the dorsolateral part of the spinal cord. These motorneurons project their fibers into the ventral root through the ventral part of the spinal cord (d).
Figure 9. GFP stained sections of DRG, counterstaned with haematoxilin. Transduction of the Dorsal root ganglia along the spinal cord can be seen. DRG from cervical (A,B), thoracic (C,D) and lumbar (E,F) levels are transduced. At a higher magnification (right puicture, B,D,F) the fibers in the root are visible through their expression of GFP. Almost all DRG neurons are transduced.
Figure 10. Double staining of the cerebellum for GFP and NeuN, a neuronal marker. The Bergmann glia cells (Bc) show GFP expression, and based on its morphology, also a Purkinje cell (Pc) can be observed.
Figure 11. Double staining of the cortex for GFP and NeuN. The arrowhead is pointing at a neuron that is expressing GFP in the cortex.
Figure 12. Double staining of the cortex for GFP and GFAP, an astrocitic marker. The arrow is pointing at an astrocyte expressing GFP. The arrowhead is pointing at a cell that has characteristics of a neuron.
Figure 13. Haematoxilin Eosin staining of sections of the brain and cerebellum. Top left is an overview of the cerebellum. Top right is a higher magnification of the molecular layer of the cerebellum. Nicely aligned are the large round neurons (see also inset), the Purkinje cells. In general, no obvious infiltrates or irregularities are observed indicating that the treatment is well tolerated.
Fig. 14. GFP stained sections of the lumbar spinal cord showing dose dependent transduction of motorneurons in the lumbar spinal cord by presence of the transgene of large neurons in the ventral horn. Animals were infused with either 5E13 (A), 5E14 (C) or 8E14 (E) genomic copies (GC) into the CSF. Higher magnifications are shown of the ventral horn, where the motorneurons are located, are depicted (B, D and F).

### Examples

### Example 1.

In this experiment, AAV-5 vectors encoding for green fluorescent protein (GFP) under control of the CAG promoter were intrathecally infused into the CSF of non-human primates. Transgene expression was examined at 4 weeks post-injection. Expression of GFP was observed in the motorneurons and the dorsal root ganglia (DRG) at all spinal levels. Moreover, in the cerebellum many Bergman glia were transduced as well as some Purkinje cells. In the brain, the cortex was also expressing GFP in both neurons and glia. Cells along the subventricular zone (SVZ) were also transduced. Altogether, these results indicate that using this route of injection, a large area of the CNS is covered and gene therapy for the CNS becomes a realistic option.

### Example 1.1. Materials and Methods

### 1.1.1. Preparation of the vector

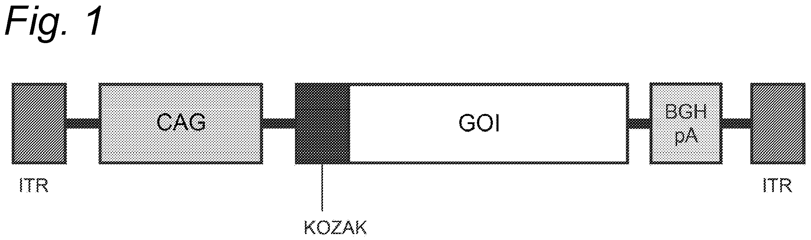

The expression cassette of rAAV serotype 5 (rAAV-5) contains the cDNA of the human the enhanced green fluorescent protein (EGFP) cDNA gene. Expression is under the control of The CAG promoter, a combination of the cytomegalovirus (CMV) early enhancer element and chicken beta-actin promoter. The GFP is preceded by a Kozak sequence and polyadenylated by the Bovine growth hormone polyadenylation (BGHpA) signal. The whole cassette is flanked by two non-coding inverted terminal repeats of AAV-2 (see Figure 1). Recombinant AAV-5 vectors were prepared using a baculovirus expression system similar as described earlier (Urabe et al., 2002, Unzu et al., 2011, reviewed in Kotin, 2011). Briefly, three recombinant baculoviruses, one encoding for the REP for replication and packaging, one encoding for the CAP-5 for the capsid of AAV-5 and one with the expression cassette, were used to infect SF9 insect cells. Purification was performed using AVB Sepharose high-performance affinity medium (GE Healthcare, Piscataway, NJ). The vectors were titered using a Q-PCR method with primer-probe combinations directed against the transgene and titers were expressed as genomic copies per ml (GC/ml). Titer of the vector was 1.4E14 GC/ml.

### 1.1.2. Animals and tissue collection

Male Cynolmolgus monkeys (Macaca fascicularis) ~4 kg were used in this study. Daily health observations and periodic body weight measurements were recorded in order to assess the welfare of the animals. Veterinarians reported no abnormal symptoms throughout the study. All procedures were approved by the Institutional Animal Care and Use Committee at Valley Biosystems (Sacramento, CA, USA). Before starting the procedure, animals were negatively tested for the presence of neutralizing antibodies against AAV (see below). For the procedure, animals were anesthetized with a mixture of ketamine (Ketaset, 7 mg/kg) and dexmedetomidine hydrochloride (Dexdomitor, 0.015 mg/kg). After surgery, all animals received an intramuscular dose of atipamezole hydrochloride to reverse anesthesia (Antisedan, 0.15 mg/kg). One months after AAV-5 administration, animals were deeply anesthetized and perfused transcardially with phosphate-buffered saline (PBS) and 4% paraformaldehyde (PFA) in PBS. Brains and organs were collected and processed for histological analysis.

Briefly, brains and cervical, thoracic and lumbar parts of the spinal cord were sliced coronally into 6-mm blocks, postfixed in 4% PFA-PBS overnight, and cryoprotected in 30% (w/v) sucrose the next day. For histological staining, a sliding microtome (Thermo Scientific Microm HM 450; Thermo Fisher Scientific, Waltham, MA) was used to cut brain blocks into 40-lm serial sections. Sections were then placed in cryoprotectant solution until use.

### 1.1.3. AAV delivery

After induction of anesthesia, the animal's head was placed in a stereotactic frame and flexed in a prone position, and the back of the neck was cleaned with povidone-iodine and alcohol. A 3-ml syringe attached to a 1-inch 23-gauge needle was mounted onto the
micromanipulator, and the needle was manually guided into the Cisterna magna. The lumbar sac was approached similarly between the spinal level of L4 and 5. Penetration was verified by aspiration of a small volume of cerebrospinal fluid (CSF) into the syringe, which was then secured to the micromanipulator. The three-way stopcock on the syringe was then adjusted to allow infusion of 3 ml of vector at 0.5 ml/min with a pump (3500 Medfusion; Strategic Applications, Libertyville, IL). After completion of the infusion, the line was flushed with 0.2 ml of saline. Last, a small volume of CSF was aspirated to verify that the needle was still in the CM. After verification, the needle was slowly removed. Hence, each animal received 3 ml into the cisterna magna and 3 ml by lumbar injection. In total, 6 ml of 1.4E14 GC/ml of AAV-5-CAG-GFP was infused.

Similarly, animals can be treated with the same amount and dosage of AAV-5-CAG-GFP, which is administered only via lumbar injection between spinal level L4-L5 (no injection into the cisterna magna). The following amounts of AAV-5-CAG-GFP were administrated via lumbar injection: 5E13 (normal dose), 5E14 and 8E14 GC (high dose).

A normal dose for AAV administration is around 1E12 GC and 5E13 GC. For example, Vulchanova (*supra*) uses 1E11 GC AAV-5 for a mouse (20g), which equals 5E12/kg. For a 5kg monkey, this constitutes 2.5E13 GC. In addition, Gray et al (2013, Gene Therapy, 20, 450 - 459) uses 2E12 GC per animal of 6 kg. Therefore, the administrated dose of 5.0E13 GC can be considered a normal dose in the art, and the doses of 5.0E14 and 8.4E14 are considered a high dose.

Since lumbar infusion of a blue dye results in blue dye that is tapped cisternally, no differences in outcome are expected if the gene therapy vector is only administered lumbar.

### 1.1.4. Immunohistochemistry

Chromogenic and immunofluorescence staining were performed on 40-lm coronal sections throughout four alternate 6-mm blocks from the prefrontal cortex to the cerebellum and the spinal cord. Chromogenic GFP staining was performed as described previously (Hadaczek et al., 2009). Briefly, sections were washed in PBS, blocked for endogenous peroxidase activity in 1% H2O2-30% ethanol in PBS, and rinsed in PBST (PBS plus 0.1% Tween 20). Sections were then incubated in Background Sniper blocking solution (BS966G; Biocare Medical, Concord, CA) and incubated for up to 24 hr at 4 °C with primary antibody against GFP (rabbit anti-GFP, diluted 1:3000; Millipore, Bedford, MA) in Da Vinci Green diluent (PD900; Biocare Medical). The next day, after being washed in PBST, sections were incubated in Rabbit Mach 3 Polymer HRP (RP531L; Biocare Medical) for GFP staining at ambient temperature and color was developed with 3,3¢-diaminobenzidine (DAB) (DAB peroxidase substrate kit, SK-4100; Vector Laboratories, Burlingame, CA). Sections were then mounted on slides, dehydrated, and coverslipped with Shandon-Mount (cat. No 1900333; Thermo Fisher Scientific).

To determine the phenotype of GFP-positive cells, double immunofluorescence staining was performed. Brain sections were washed with PBST, blocked for 60 min in 20% normal horse serum (NHS; Jackson ImmunoResearch, West Grove, PA), and then incubated overnight at 4_C with anti-GFP (rabbit or mouse, diluted 1:200; Millipore) together with antibodies specific for either microglia (anti-lba1, rabbit polyclonal, diluted 1:500; Biocare Medical), neurons (anti-NeuN, mouse monoclonal, diluted 1:300; Millipore), fibrous astrocytes (anti-glial-fibrillary acidic protein [GFAP], mouse
monoclonal, diluted 1:500; Dako, Carpinteria, CA). All antibodies were diluted in Da Vinci Green diluent (PD900; Biocare Medical). After incubation with primary antibodies, sections were washed in PBST; incubated with a cocktail of corresponding secondary antibodies (FITC-conjugated anti-rabbit [diluted 1 :200; Jackson ImmunoResearch] or Alexa 488-conjugated anti-mouse [diluted 1:500; Molecular Probes/Invitrogen, Carlsbad, CA] and Alexa 555-conjugated anti-mouse [diluted 1:500; Molecular Probes/lnvitrogen] or Alexa 555- conjugated anti-rabbit [diluted 1:500; Molecular Probes/lnvitrogen]) in PBST for 2 hr at room temperature; washed in PBS; and wet-mounted on frosted slides. Sections were coverslipped with a 4¢,6-diamidino-2-phenylindole (DAPI)- containing hard-set media to identify all nuclei (Vectashield H-1200; Vector Laboratories).

### 1.1.5. Anti-AAV5 antibody titer in NHPs

The titer of antibodies directed against AAV capsid was determined by ELISA on blood samples before vector administration. Briefly, a 2 E 10 GC/ml solution of AAV5 capsids in 1mM carbonate buffer was distributed into a 96-well titer plate and incubated overnight at 4 °C. The next day, the plate was washed and blocked with a 5% nonfat milk solution in PBST. Sera were diluted over a range from 1:50 to 1:6400 and incubated at room temperature for 1 hr. Wells were then washed with PBST and incubated with a horseradish peroxidase (HRP)-conjugated anti-monkey secondary antibody (Sigma-Aldrich, St. Louis, MO) at room temperature for 1 hr. The wells were washed with again in PBST and then developed with 3,3-,5,5-tetramethylbenzidine (TMB). The reaction was stopped by the addition of hydrochloric acid and absorbance was read at 450nm on a plate reader (Bevan et al., 2011).

### 1.1.6 Biodistribution AA Vs

Pre-Dose: Blood samples were collected from adult cynomolgus macaques (Macaca fasciularis) for screening and analyzed for serum anti-AAV-5 antibodies titer, serum clinical chemistry and hematology evaluations.

CSF Infusion: Animals were immobilized with ketamine (10 mg/kg) IM and medetomidine (15 µg/kg) IM. A spinal needle was placed in the lumbar region where the animal is injected with a single dose (consisting of 5 mL over 5 min) of AAV5-CAG-GFP into the CSF. Three different doses were administered in these animals (5E14, 5E13 and 5E12 GC total).

Tissue harvesting: The CSF-infused animals were euthanized at 8 weeks post-infusion. Each animal was deeply anesthetized with ketamine (10 to 15 mg/kg, IM) and medetomidine (15 µg/kg). The animals were dosed with euthanasia solution. Following verification of a deep plane of anesthesia (i.e. lack of toe pinch response, corneal reflexes and jaw tone), a trans-thoracotomy was performed and a piece of the liver sampled. The brain and spinal cord were harvested and a tissue punch was taken for molecular (Q-PCR) analysis. The remainder of the tissue was saved in paraformaldehyde for histological evaluations.

Q-PCR analysis: Tissue was subjected to a standard Q-PCR method with primers directed against the GFP gene. The outcome is depicted in table 2.

### 1.2. Results

The procedure was well tolerated by the animals. No weight loss was observed and also no clinical signs were noted by the veterinarian.

Three different dosages of AAV were tested: 5E13 GC, 5E14 GC and 8.4E14 GC. The 5E13 GC can be considered a regular dose, while 5E14 GC and 8.4E14 GC constitute high dosages.

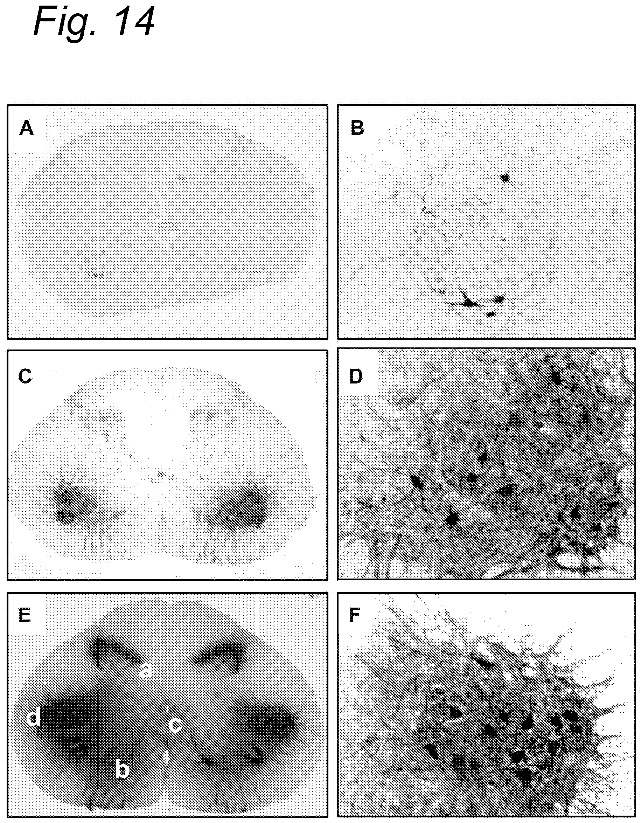

As indicated in figure 14, administration of a regular AAV5 dose (5E13 GC, which is a reference example) resulted in hardly any transduction, confirming the consensus in the art that AAV5 only poorly transduces neuronal cells following intrathecal administration. Surprisingly, higher doses of AAV5 resulted in a significant transduction of motorneurons. Figure 14 shows that there is an increasing transduction of motorneurons when a higher dose is used. In the dorsal horn, some fibers are also observed, suggesting transduction of DRG neurons. However, the predominant transduced cell type constitute motor neurons.

Furthermore, the presence of AAV genomes was determined in the spinal cord, brain and liver. In particular, the presence of AAV in the liver would indicate that there is leakage of AAV5 from the CNS into the periphery. It is well-known that AAV vectors can leak into the periphery upon administration into the CNS. For example, Haurigot et al (2013, J. Of Clin. Invest., 123:3254 - 3271) is using AAV9 for complete body correction with intracranial injection that also serves the liver. Interestingly, in contrast to e.g. AAV9, most of the AAV5 particles remain within the spinal cord, with spread to the brain. The liver is not invaded by AAV5 (Table 2).

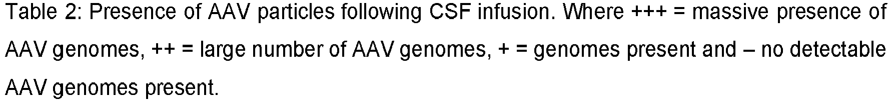

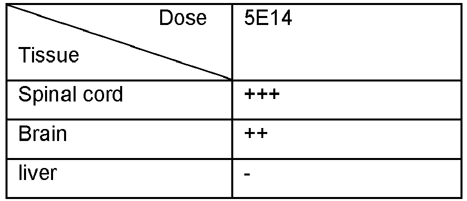

The following observations derive from experiments performed with 8.4E14 GC: GFP expression was observed throughout the whole CNS, including brain, cerebellum and spinal cord. Expression was observed in both neuronal and glial cells. In particular, GFP expression was predominantly observed in the motor neurons. Vulchanova et al also observe transduction of neuronal cells with AAV5, after pretreatment with mannitol. However, Vulchanova et al see a much higher transduction efficiency of DRG neurons (and their neurites projecting into the dorsal horn of the spinal cord) in comparison the motor neurons. Thus, the ratio of transduced motor neurons/DRG would be in favour of the DRG with mannitol and for motor neurons with a high dose of AAV5, making the administration of a high dose of AAV5 particularly suitable for treatment and/or prevention of motor neuron-related conditions.

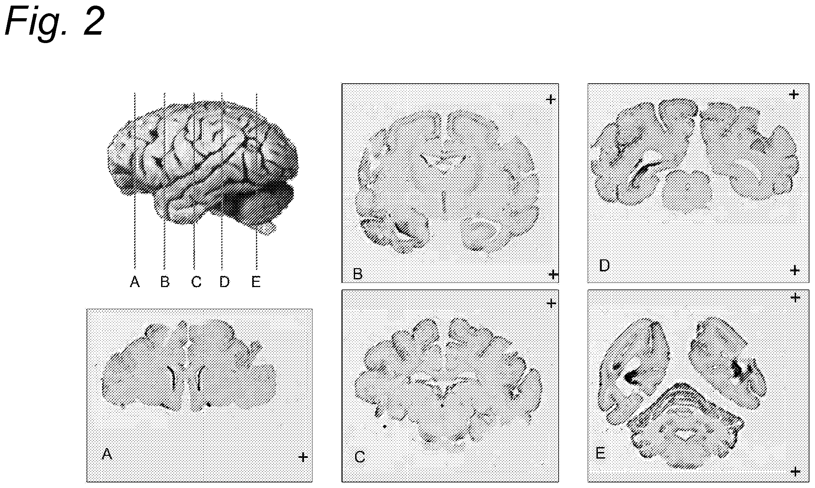

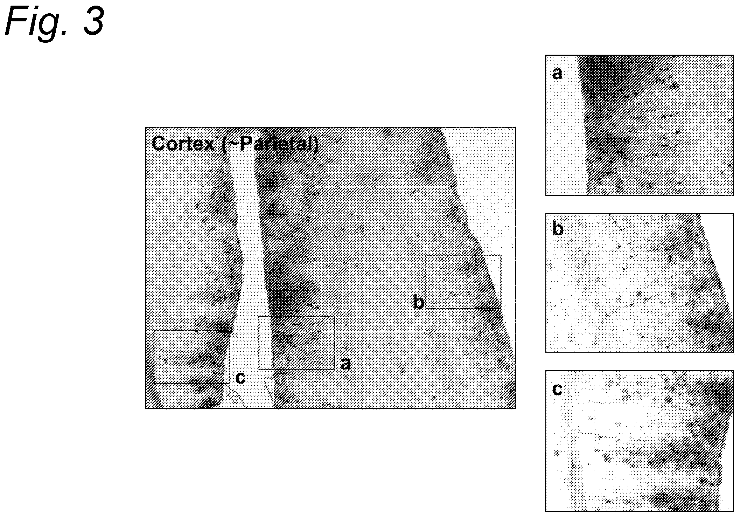

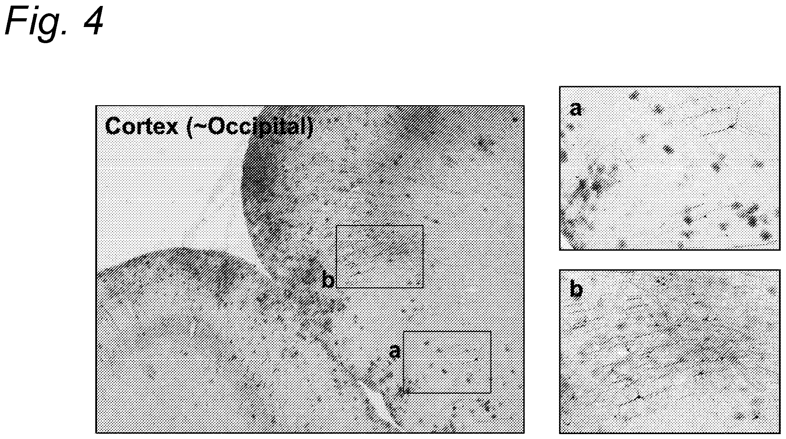

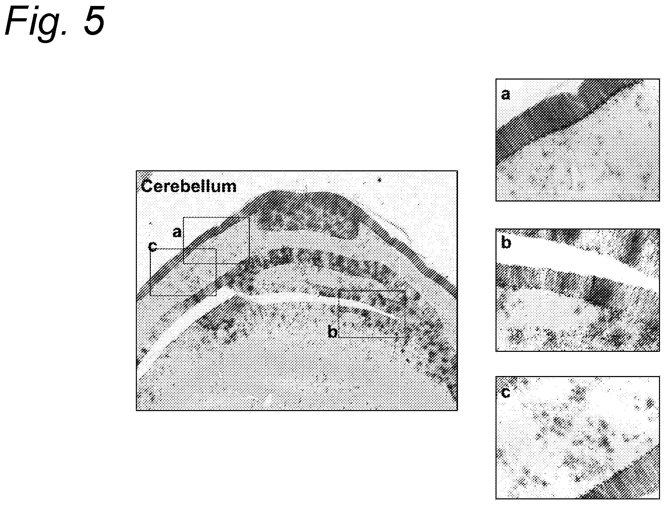

An overview several slices of the brain showed nice expression throughout the whole brain, mainly in relative close vicinity of the ventricle walls (see Figure 2). Expression in the parietal cortex showed expression through several layers of the cortex. Also some astrocytes were transduced (Figure 3). The same holds true for the occipital part of the cortex (Figure 4). In the cerebellum, the windings of the structure of the cerebellum were clearly depicted by the line of transduced cells (Figure 5). Also in this part of the brain, both neuronal and glial cell transduction is observed.

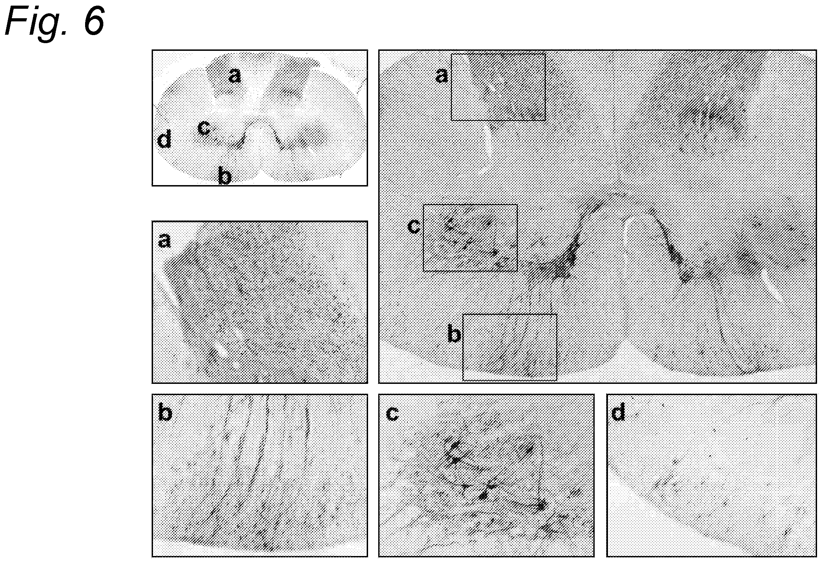

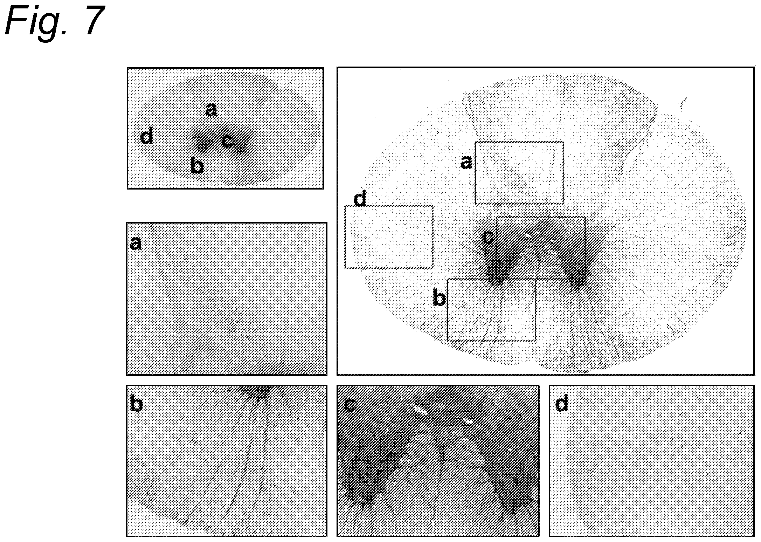

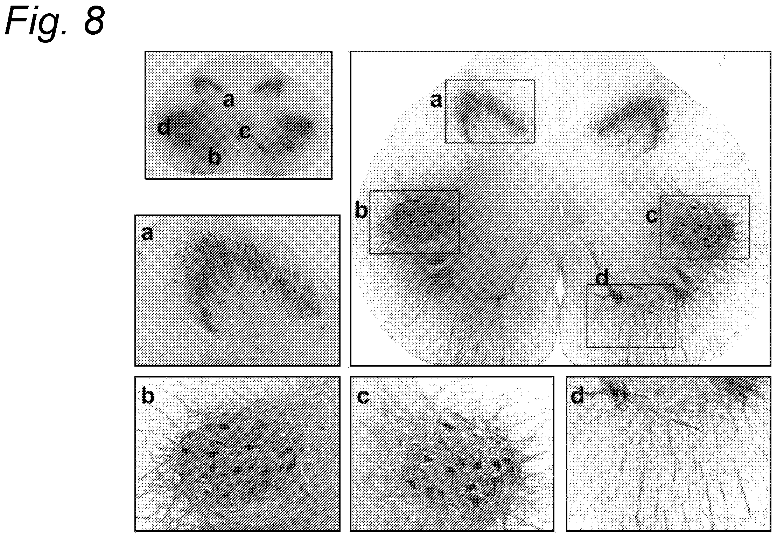

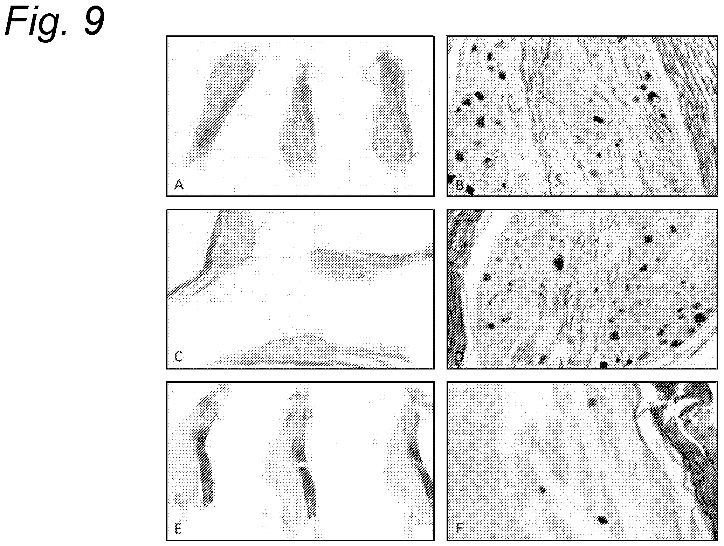

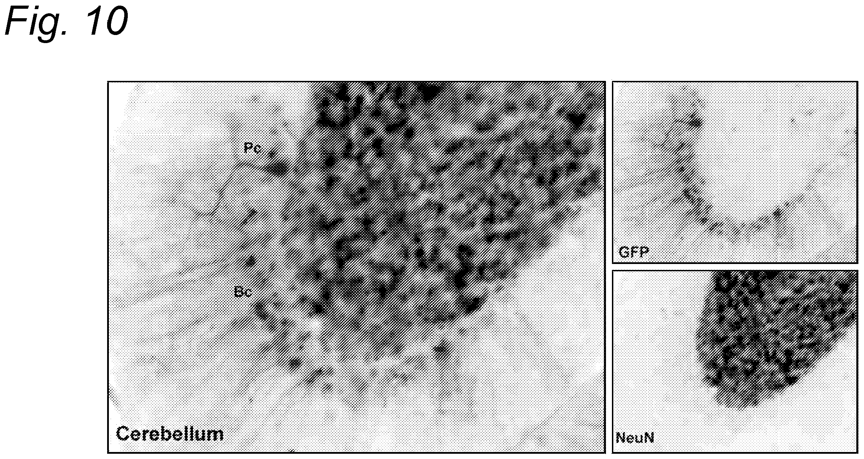

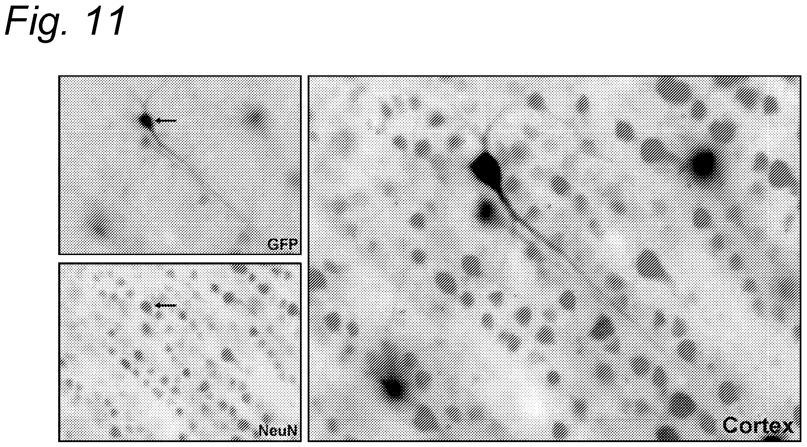

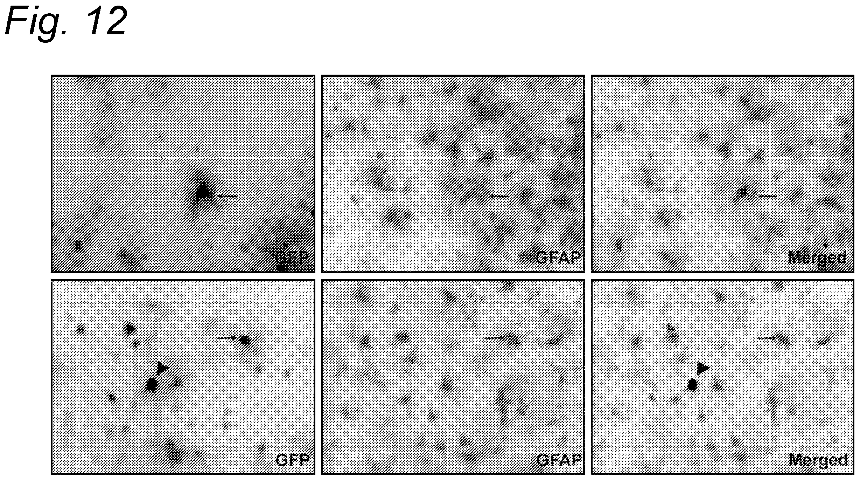

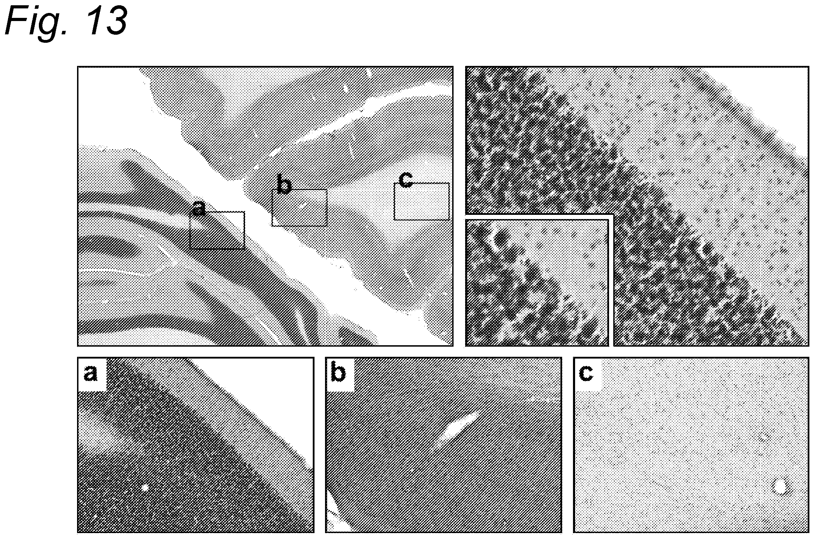

In the spinal cord, many (if not all) motorneurons were transduced over the entire length of the spinal cord (Figure 6, 7 and 8). A similar observation was made for the neurons in the DRG (Figure 9). They were also heavily transduced to express GFP. To identify the cells that were expressing the transgene, double staining for neuronal and astrocytic markers was performed. Using this specific CAG promoter, bot neuronal and astrocytic transduction was achieved (Figures 10, 11 and 12). To examine whether neuronal loss had occurred, H&E staining was performed on sections of the cerebellum (Figure 13). Based on the remaining presence of the Purkinje cell layer, no neurotoxicity was observed using this specific approach.

### 1.3. Discussion

The delivery of a gene to the CNS has so far been hampered by the size and complexity of the CNS. Following a single injection into the brain, expression of the transgene is local and remains mainly restricted to the infused area. Using the CSF as a method for delivery of the viral vector, a larger area can be reached. The current study shows that using an AAV-5 vector at a relatively high dose of over 1E14 GC/kg, it is possible to reach this goal of transduction over a larger area in the CNS. The treatment was well tolerated as no clinical signs and no obvious neuronal loss were observed. Both neuronal and glial cells were transduced using this specific CAG promoter. Using cell specific promoters, such as the GFAP promoter or synapsin-1 promoter, one could direct the expression to a specific population. We have shown that areas of the cortex, cerebellum and subventricular zone show expression of the transgene in both neurons and glial cells. In the spinal cord, motorneuron transduction was prevalent as well as transduction of neurons in the dorsal root ganglia. These results show that the method of CNS-mediated delivery can be used to deliver genes for a gene therapeutic approach. Indications that could be helped by this could be: Motorneuron diseases such as amyotrofic laterale sclerose (ALS), spinal muscular atrophy (SMA), or sensory related indications such as pain. Other neurological indications that could be helped by such an approach are for instance Huntington's disease, Alzheimer's disease, Tay-Says disease, Friedreich ataxia, ataxia telangietacsia, Spinocerebellar ataxia (type 1, 2 and 3), Niemann-Pick disease (A, B and C), Dopa-responsive dystonia, Fragile X syndrome, Krabbe disease, Glycogen storage disease type 2 (Pompe), Primary lateral sclerosis, Pelizaeus-Merzbacher disease, X-linked adrenoleukodystrophy, Giant axonal neuropathy, Multiple system atrophy (MSA), Proximal myotonic myopathy, and Neuronal Ceroid Lipofuscinosis (Batten disease).

The genetic defects that are responsible are largely known and could be built into an AAV(-5) vector. Using this delivery system as a therapeutic agent, one could imagine that this reverses the genotype and hence hopefully also the phenotype.

Moreover, the intrathecal method of delivery is regarded as less invasive as infusion into the CNS itself as brain surgery requires complex surgery. The intrathecal approach has as additional value that this could be performed without the need of a specialized center for brain injections.

### 1.4. References

Bantel-Schaal U1, Delius H, Schmidt R, zur Hausen H. J Virol. 1999 Feb;73(2):939-47. Human adeno-associated virus type 5 is only distantly related to other known primate helper-dependent parvoviruses.
Bartlett JS1, Samulski RJ, McCown TJ. Hum Gene Ther. 1998 May 20;9(8):1181-6. Selective and rapid uptake of adeno-associated virus type 2 in brain.
Bevan AK, Duque S, Foust KD, Morales PR, Braun L, Schmelzer L, Chan CM, McCrate M, Chicoine LG, Coley BD, Porensky PN, Kolb SJ, Mendell JR, Burghes AH, Kaspar BK. Systemic gene delivery in large species for targeting spinal cord, brain, and peripheral tissues for pediatric disorders. Mol Ther. 2011 Nov;19(11):1971-80.
Blits B1, Derks S, Twisk J, Ehlert E, Prins J, Verhaagen J. J Neurosci Methods. 2010 Jan 15;185(2):257-63. Adeno-associated viral vector (AAV)-mediated gene transfer in the red nucleus of the adult rat brain: comparative analysis of the transduction properties of seven AAV serotypes and lentiviral vectors.
Chiorini JA1, Yang L, Liu Y, Safer B, Kotin RM. J Virol. 1997 Sep;71(9):6823-33. Cloning of adeno-associated virus type 4 (AAV4) and generation of recombinant AAV4 particles.
Data Standards Manual. "Route of Administration". Food and Drug Administration. Retrieved 11 March 2011).
Davidson BL 1, Stein CS, Heth JA, Martins I, Kotin RM, Derksen TA, Zabner J, Ghodsi A, Chiorini JA. Proc Natl Acad Sci USA. 2000 Mar 28;97(7):3428-32. Recombinant adeno-associated virus type 2, 4, and 5 vectors: transduction of variant cell types and regions in the mammalian central nervous system.
Donello JE1, Loeb JE, Hope TJ. J Virol. 1998 Jun;72(6):5085-92. Woodchuck hepatitis virus contains a tripartite posttranscriptional regulatory element.
Drinkut A1, Tereshchenko Y, Schulz JB, Bähr M, Kügler S. Mol Ther. 2012 Mar;20(3):534-43. Efficient gene therapy for Parkinson's disease using astrocytes as hosts for localized neurotrophic factor delivery.
Finegold AA1, Mannes AJ, ladarola MJ. Hum Gene Ther. 1999 May 1;10(7):1251-7. A paracrine paradigm for in vivo gene therapy in the central nervous system: treatment of chronic pain.
Goncalves, 2005, Virol J. 2(1):43 Tropism-modified adenoviral and adeno-associated viral vectors for gene therapy.
Gossen M1, Bujard H. Proc Natl Acad Sci USA. 1992 Jun 15;89(12):5547-51. Tight control of gene expression in mammalian cells by tetracycline-responsive promoters.
Gray SJ1, Nagabhushan Kalburgi S, McCown TJ, Jude Samulski R. Gene Ther. 2013 Apr;20(4):450-9. doi: 10.1038/gt.2012.101. Epub 2013 Jan 10. Global CNS gene delivery and evasion of anti-AAV-neutralizing antibodies by intrathecal AAV administration in non-human primates.
Grimm D1, Kleinschmidt JA. Hum Gene Ther. 1999 Oct 10;10(15):2445-50. Progress in adeno-associated virus type 2 vector production: promises and prospects for clinical use.
Grimm D1, Kay MA, Kleinschmidt JA. Mol Ther. 2003 Jun;7(6):839-50. Helper virus-free, optically controllable, and two-plasmid-based production of adeno-associated virus vectors of serotypes 1 to 6.
Hadaczek P1, Forsayeth J, Mirek H, Munson K, Bringas J, Pivirotto P, McBride JL, Davidson BL, Bankiewicz KS. Hum Gene Ther. 2009 Mar;20(3):225-37. Transduction of nonhuman primate brain with adeno-associated virus serotype 1: vector trafficking and immune response. Jennings K1, Miyamae T, Traister R, MarinovA, Katakura S, Sowders D, Trapnell B, Wilson JM, Gao G, Hirsch R. Mol Ther. 2005 Apr;11(4):600-7. Proteasome inhibition enhances AAV-mediated transgene expression in human synoviocytes in vitro and in vivo.
Kaplitt MG1, Leone P, Samulski RJ, Xiao X, Pfaff DW, O'Malley KL, During MJ. Nat Genet. 1994 Oct;8(2):148-54. Long-term gene expression and phenotypic correction using adeno-associated virus vectors in the mammalian brain.
Kenneth I. Berns, "Parvoviridae: The Viruses and Their Replication," Chapter 69 in Fields Virology (3d Ed. 1996).
Klein RL1, Meyer EM, Peel AL, Zolotukhin S, Meyers C, Muzyczka N, King MA. ), Exp Neurol. 1998 Apr;150(2):183-94. Neuron-specific transduction in the rat septohippocampal or nigrostriatal pathway by recombinant adeno-associated virus vectors.
Klein RL1, Dayton RD, Tatom JB, Henderson KM, Henning PP. Mol Ther. 2008 Jan;16(1):89-96. Epub 2007 Oct 23. AAV8, 9, Rh10, Rh43 vector gene transfer in the rat brain: effects of serotype, promoter and purification method.
Kotin RM. Large-scale recombinant adeno-associated virus production. Hum Mol Genet. 2011 Apr 15;20(R1):R2-6.
Kügler S1, Meyn L, Holzmüller H, Gerhardt E, Isenmann S, Schulz JB, Bähr M. Mol Cell Neurosci. 2001 Jan;17(1):78-96. Neuron-specific expression of therapeutic proteins: evaluation of different cellular promoters in recombinant adenoviral vectors.
Lee Y1, Messing A, Su M, Brenner M. Glia. 2008 Apr;56(5):481-93. GFAP promoter elements required for region-specific and astrocyte-specific expression.
Lin CR1, Yang LC, Lee TH, Lee CT, Huang HT, Sun WZ, Cheng JT. Gene Ther. 2002 Sep;9(18):1247-53. Electroporation-mediated pain-killer gene therapy for mononeuropathic rats.
Lin CR1, Tai MH, Cheng JT, Chou AK, Wang JJ, Tan PH, Marsala M, Yang LC. Neurosci Lett. 2002 Jan 4;317(1):1-4. Electroporation for direct spinal gene transfer in rats.
Maddalena et al.(2013) Molecular Therapy - Nucleic Acids 2:e106
Maddalena A1, Tereshchenko J, Bähr M, Kügler S. Mol Ther Nucleic Acids. 2013 Jul 16;2:e106. Adeno-associated Virus-mediated, Mifepristone-regulated Transgene Expression in the Brain. Mandel RJ1, Rendahl KG, Spratt SK, Snyder RO, Cohen LK, Leff SE. J Neurosci. 1998 Jun 1;18(11):4271-84. Characterization of intrastriatal recombinant adeno-associated virus-mediated gene transfer of human tyrosine hydroxylase and human GTP-cyclohydrolase I in a rat model of Parkinson's disease.
McCarty DM. Mol Ther. 2008 Oct;16(10):1648-56. Self-complementary AAV vectors; advances and applications.
Milligan ED1, Langer SJ, Sloane EM, He L, Wieseler-Frank J, O'Connor K, Martin D, Forsayeth JR, Maier SF, Johnson K, Chavez RA, Leinwand LA, Watkins LR. Eur J Neurosci. 2005 Apr;21(8):2136-48. Controlling pathological pain by adenovirally driven spinal production of the anti-inflammatory cytokine, interleukin-10.
Milligan ED1, Sloane EM, Langer SJ, Cruz PE, Chacur M, Spataro L, Wieseler-Frank J, Hammack SE, Maier SF, Flotte TR, Forsayeth JR, Leinwand LA, Chavez R, Watkins LR. Mol Pain. 2005 Feb 25;1:9. Controlling neuropathic pain by adeno-associated virus driven production of the anti-inflammatory cytokine, interleukin-10.
Milligan ED1, Sloane EM, Langer SJ, Hughes TS, Jekich BM, Frank MG, Mahoney JH, Levkoff LH, Maier SF, Cruz PE, Flotte TR, Johnson KW, Mahoney MM, Chavez RA, Leinwand LA, Watkins LR. Pain. 2006 Dec 15;126(1-3):294-308. Repeated intrathecal injections of plasmid DNA encoding interleukin-10 produce prolonged reversal of neuropathic pain.
Miyazaki J1, Takaki S, Araki K, Tashiro F, Tominaga A, Takatsu K, Yamamura K. Gene. 1989 Jul 15;79(2):269-77. Expression vector system based on the chicken beta-actin promoter directs efficient production of interleukin-5.
Muyldermans S. J Biotechnol. 2001 Jun;74(4):277-302. Single domain camel antibodies: current status.
Passini MA 1, Lee EB, Heuer GG, Wolfe JH. J Neurosci. 2002 Aug 1;22(15):6437-46. Distribution of a lysosomal enzyme in the adult brain by axonal transport and by cells of the rostral migratory stream.
Russell WC. J Gen Virol. 2000 Nov;81(Pt 11):2573-604. Update on adenovirus and its vectors. Rutledge EA1, Halbert CL, Russell DW. J Virol. 1998 Jan;72(1):309-19. Infectious clones and vectors derived from adeno-associated virus (AAV) serotypes other than AAV type 2. Sambrook and Russell (2001) "Molecular Cloning: A Laboratory Manual (3rd edition), Cold Spring Harbor Laboratory, Cold Spring Harbor Laboratory Press, New York.
Shevtsova Z1, Malik JM, Michel U, Bähr M, Kügler S. Exp Physiol. 2005 Jan;90(1):53-9. Promoters and serotypes: targeting of adeno-associated virus vectors for gene transfer in the rat central nervous system in vitro and in vivo.
Shipley JM1, Miller RD, Wu BM, Grubb JH, Christensen SG, Kyle JW, Sly WS. Genomics. 1991 Aug;10(4):1009-18. Analysis of the 5' flanking region of the human beta-glucuronidase gene. Srivastava A, Lusby EW, Berns KI. J Virol. 1983 Feb;45(2):555-64. Nucleotide sequence and organization of the adeno-associated virus 2 genome.
Storek B1, Reinhardt M, Wang C, Janssen WG, Harder NM, Banck MS, Morrison JH, Beutler AS. Proc Natl Acad Sci USA. 2008 Jan 22;105(3):1055-60. Sensory neuron targeting by self-complementary AAV8 via lumbar puncture for chronic pain.
Tratschin JD, Miller IL, Smith MG, Carter BJ. Mol Cell Biol. 1985 Nov;5(11):3251-60. Adeno-associated virus vector for high-frequency integration, expression, and rescue of genes in mammalian cells.
Unzu C, Sampedro A, Mauleón I, Alegre M, Beattie SG, de Salamanca RE, Snapper J, Twisk J, Petry H, González-Aseguinolaza G, Artieda J, Rodriguez-Pena MS, Prieto J, Fontanellas A. Sustained enzymatic correction by rAAV-mediated liver gene therapy protects against induced motor neuropathy in acute porphyria mice. Mol Ther. 2011 Feb;19(2):243-50.
Urabe M1, Nakakura T, Xin KQ, Obara Y, Mizukami H, Kume A, Kotin RM, Ozawa K. J Virol. 2006 Feb;80(4):1874-85. Scalable generation of high-titer recombinant adeno-associated virus type 5 in insect cells.
Urabe, M, Ding, C and Kotin, RM (2002). Insect cells as a factory to produce adeno associated virus type 2 vectors. Hum Gene Ther 13: 1935-1943.
US20080008690: AAV virions with decreased immunoreactivity and uses therefor
Vulchanova L1, Schuster DJ, Belur LR, Riedl MS, Podetz-Pedersen KM, Kitto KF, Wilcox GL, Mclvor RS, Fairbanks CA. Mol Pain. 2010 May 28;6:31. doi: 10.1186/1744-8069-6-31. Differential adeno-associated virus mediated gene transfer to sensory neurons following intrathecal delivery by direct lumbar puncture.
WO 2007/046703: Improved aav vectors produced in insect cells
WO 2007/148971: Vectors with modified initiation codon for the translation of aav-rep78 useful for production of aav in insect cells
WO 2009/014445: Baculoviral vectors comprising repeated coding sequences with differential codon biases
WO 2009/104964: Optimisation of expression of parvoviral rep and cap proteins in insect cells
WO 2011/112089: Mutated rep encoding sequences for use in aav production
WO 2013/036118: Removal of contaminating viruses from aav preparations
WO2001/092551: Duplexed parvovirus vectors
WO2011/122950: Monomeric duplex aav vectors
Wu P1, Xiao W, Conlon T, Hughes J, Agbandje-McKenna M, Ferkol T, Flotte T, Muzyczka N. J Virol. 2000 Sep;74(18):8635-47. Mutational analysis of the adeno-associated virus type 2 (AAV2) capsid gene and construction of AAV2 vectors with altered tropism.
Xu R1, Janson CG, Mastakov M, Lawlor P, Young D, Mouravlev A, Fitzsimons H, Choi KL, Ma H, Dragunow M, Leone P, Chen Q, Dicker B, During MJ. Gene Ther. 2001 Sep;8(17):1323-32. Quantitative comparison of expression with adeno-associated virus (AAV-2) brain-specific gene cassettes.
Zaldumbide A1, Hoeben RC. Gene Ther. 2008 Feb;15(4):239-46. How not to be seen: immuneevasion strategies in gene therapy.

## Claims

1. An adeno-associated virus (AAV) gene therapy vector for use as a medicament for treating CNS disorders in a mammalian subject, wherein the mammalian subject is human, wherein the gene therapy vector comprises AAV serotype 5 capsid proteins and a gene product of interest flanked by AAV inverted terminal repeats (ITRs) and wherein the gene product of interest is operably linked to expression control elements comprising a promoter that produces sufficient expression of the gene product of interest to obtain a therapeutic effect, and wherein the gene therapy vector is administered by intrathecal lumbar administration, wherein 1 × 10¹⁴ - 1 × 10¹⁶ genome copies per kilogram of body weight is administered to the subject.

2. An AAV gene therapy vector for use according to claim 1, wherein the intrathecal lumbar administration is at a position selected from the group consisting of: L4 - L5, L3 - L4, L1 - L2 and L2 - L3.

3. An AAV gene therapy vector for use according to claim 1 or claim 2, wherein the AAV gene therapy vector is a single stranded AAV gene therapy vector or a monomeric duplex vector.

4. An AAV gene therapy vector for use according to any one of the preceding claims, wherein the gene product of interest is for use in the treatment or prophylaxis of a condition selected from the group consisting of: amyotrophic lateral sclerosis (ALS), spinal muscular atrophy (SMA), pain, Huntington's disease, Alzheimer's disease, Tay- Says disease, Friedreich ataxia, ataxia telangiectasia, Spinocerebellar ataxia type 1, 2 and 3, Niemann-Pick disease A, B and C, Dopa-responsive dystonia, Fragile X syndrome, Krabbe disease, Glycogen storage disease type 2 (Pompe), Primary lateral sclerosis, Pelizaeus-Merzbacher disease, X-linked adrenoleukodystrophy, Giant axonal neuropathy, Multiple system atrophy (MSA), Proximal myotonic myopathy, Neuronal Ceroid Lipofuscinosis (Batten disease) and cancer.

5. An AAV gene therapy vector for use according to any one of the preceding claims, wherein the AAV ITRs are AAV serotype 2 ITRs.

6. An AAV gene therapy vector for use according to any one of the preceding claims, wherein the gene product of interest is selected from the group consisting of: Aspartylglucosaminidase, α-Galactosidase A, Palmitoyl Protein Thioesterase, Tripeptidyl Peptidase, Lysosomal Transmembrane Protein, Multiple gene products, Cysteine transporter, Acid ceramidase, Acid α-L-fucosidase, Protective protein/cathepsin A, Acid β-glucosidase, or glucocerebrosidase, Acid β-galactosidase, Iduronate-2-sulfatase, α-L-Iduronidase, Galactocerebrosidase, Acid α-mannosidase, Acid β-mannosidase, Arylsulfatase B, Arylsulfatase A, N-Acetylgalactosamine-6-sulfate sulfatase, Acid β-galactosidase, N-Acetylglucosamine-I-phosphotransferase, Acid sphingomyelinase, NPC-1, Acid α-glucosidase, β-Hexosaminidase B, Heparan N-sulfatase, α-N-Acetylglucosaminidase, Acetyl-CoA:α-glucosaminide N- acetyltransferase, N-Acetylglucosamine-6-sulfate sulfatase, α-N-Acetylgalactosaminidase, α-N-Acetylgalactosaminidase, α-Neuramidase, β- Glucuronidase, β-Hexosaminidase A, Acid Lipase, neurotrophic factors such as Nerve Growth Factor (NGF), Neurotrophin-3 (NT-3), Neurotrophin-4/5 (NT-4/5), Brain derived neurotrophic factor (BDNF), Cerebral Dopamine Neurotrophic factor (CDNF), glial cell line-derived neurotrophic factor (GDNF), ciliary neurotrophic factor (CNTF), growth factor Insuline-like growth factor (IGF-1), and miRNA for downregulation of a faulty gene.

7. An AAV gene therapy vector for use according to any one of the preceding claims, wherein the gene product of interest is for use in the treatment or prophylaxis of a condition associated with motor neurons and/or neurons in the dorsal root ganglia (DRGs).

8. An AAV gene therapy vector for use according to any one of the preceding claims, wherein the promoter preferably is selected from the group consisting of: cytomegalovirus (CMV) promoter, phosphoglycerate kinase (PGK), CAG promoter (a combination of the cytomegalovirus early enhancer element and chicken beta-actin promoter), glial fibrillary acidic protein (GFAP) promoter, synapsin-1 promoter, Neuron Specific Enolase (NSE) and inducible promoters such as Gene Switch or a tet-operon derived promoter.

9. An AAV gene therapy vector for use according to any one of the preceding claims, wherein the gene therapy vector is further administered into the Cisterna magna prior to, simultaneous or after the intrathecal lumbar administration.

10. An AAV gene therapy vector for use according to any one of the preceding claims, wherein 1 × 10¹⁴- 2 × 10¹⁵ genome copies per kilogram of body weight is administered to the subject.

11. An AAV gene therapy vector for use according to claim 10, wherein 1 × 10¹⁴- 6 × 10¹⁴ genome copies per kilogram of body weight is administered to the subject.

12. An AAV gene therapy vector for use according to any one of the preceding claims, wherein the subject is not subjected to pretreatment with a substance that controls intracranial pressure and/or can disrupt the intercellular tight junctions between endothelial cells of the CNS microvasculature and/or facilitates delivery of drugs such as chemotherapeutics to the CNS.

13. An AAV gene therapy vector for use according to any one of the preceding claims, wherein the subject is not subjected to intravenous mannitol pretreatment prior to intrathecal lumbar administration of the AAV gene therapy vector.

14. An AAV gene therapy vector for use according to any one of the preceding claims, wherein the AAV gene therapy vector is not a self-complementary gene vector.

## Patentansprüche

1. AAV(Adeno-assoziiertes Virus)-Gentherapievektor zur Verwendung als Medikament zur Behandlung von ZNS-Erkrankungen bei einem Säugetier, wobei das Säugetier ein Mensch ist, wobei der Gentherapievektor AAV-Serotyp-5-Kapsidproteine und ein Genprodukt von Interesse umfasst, das von AAV-invertierten terminalen Wiederholungen (ITRs) flankiert wird, und wobei das Genprodukt von Interesse funktionell mit Expressionskontrollelementen verbunden ist, die einen Promotor umfassen, der eine ausreichende Expression des Genprodukts von Interesse erzeugt, um eine therapeutische Wirkung zu erhalten, und wobei der Gentherapievektor durch intrathekale lumbale Verabreichung verabreicht wird, wobei dem Patienten 1 × 10¹⁴ - 1 × 10¹⁶ Genomkopien pro Kilogramm Körpergewicht verabreicht werden.

2. AAV-Gentherapievektor zur Verwendung nach Anspruch 1, wobei die intrathekale lumbale Verabreichung an einer Stelle erfolgt, die aus der Gruppe ausgewählt ist, die aus L4 - L5, L3 - L4, L1 - L2 und L2 - L3 besteht.

3. AAV-Gentherapievektor zur Verwendung nach Anspruch 1 oder Anspruch 2, wobei der AAV-Gentherapievektor ein einzelsträngiger AAV-Gentherapievektor oder ein monomerer Duplexvektor ist.

4. AAV-Gentherapievektor zur Verwendung nach einem der vorhergehenden Ansprüche, wobei das Genprodukt von Interesse zur Verwendung bei der Behandlung oder Prophylaxe eines Zustands bestimmt ist, der aus der Gruppe ausgewählt ist, die aus Folgendem besteht: amyotropher Lateralsklerose (ALS), spinaler Muskelatrophie (SMA), Schmerzen, Huntington-Krankheit, Alzheimer-Krankheit, Tay-Sachs-Krankheit, Friedreich-Ataxie, Ataxia teleangiectasia, spinozerebellärer Ataxie Typ 1, 2 und 3, Niemann-Pick-Krankheit A, B und C, Dopa-responsiver Dystonie, Fragilem-X-Syndrom, Krabbe-Krankheit, Glykogenspeicherkrankheit Typ 2 (Pompe), primärer Lateralsklerose, Pelizaeus-Merzbacher-Krankheit, X-chromosomaler Adrenoleukodystrophie, Riesenaxon-Neuropathie, multipler Systematrophie (MSA), proximaler myotonischer Myopathie, neuronaler Ceroid-Lipofuszinose (Batten-Krankheit) und Krebs.

5. AAV-Gentherapievektor zur Verwendung nach einem der vorhergehenden Ansprüche, wobei die AAV-ITRs AAV-Serotyp-2-ITRs sind.

6. AAV-Gentherapievektor zur Verwendung nach einem der vorhergehenden Ansprüche, wobei das Genprodukt von Interesse aus der Gruppe ausgewählt ist, die aus Folgendem besteht: Aspartylglucosaminidase, α-Galactosidase A, Palmitoyl-Protein-Thioesterase, Tripeptidyl-Peptidase, lysosomalem Transmembranprotein, multiplen Genprodukten, Cysteintransporter, saurer Ceramidase, saurer α-L-Fucosidase, Schutzprotein/Cathepsin A, saurer β-Glucosidase oder Glucocerebrosidase, saurer β-Galactosidase, Iduronat-2-Sulfatase, α-L-Iduronidase, Galactocerebrosidase, saurer α-Mannosidase, saurer β-Mannosidase, Arylsulfatase B, Arylsulfatase A, N-Acetylgalactosamin-6-sulfat-Sulfatase, saurer β-Galactosidase, N-Acetylglucosamin-I-Phosphotransferase, saurer Sphingomyelinase, NPC-1, saurer α-Glucosidase, β-Hexosaminidase B, Heparan-N-Sulfatase, α-N-Acetylglucosaminidase, Acetyl-CoA:α-Glucosaminid-N-Acetyltransferase, N-Acetylglucosamin-6-sulfat-Sulfatase, α-N-Acetylgalactosaminidase, α-N-Acetylgalactosaminidase, α-Neuramidase, β-Glucuronidase, β-Hexosaminidase A, saurer Lipase, neurotrophen Faktoren wie zum Beispiel Nervenwachstumsfaktor (NGF), Neurotrophin-3 (NT-3), Neurotrophin-4/5 (NT-4/5), aus dem Gehirn stammender neurotropher Faktor (BDNF), zerebraler dopamin-neurotropher Faktor (CDNF), aus der Glialzelle stammender neurotropher Faktor (GDNF), ziliärer neurotropher Faktor (CNTF), Wachstumsfaktor Insulin-ähnlicher Wachstumsfaktor (IGF-1) und miRNA zur Herunterregulierung eines fehlerhaften Gens.

7. AAV-Gentherapievektor zur Verwendung nach einem der vorhergehenden Ansprüche, wobei das Genprodukt von Interesse zur Verwendung bei der Behandlung oder Prophylaxe eines Zustands dient, der mit Motorneuronen und/oder Neuronen in den dorsalen Wurzelganglien (DRGs) assoziiert ist.

8. AAV-Gentherapievektor zur Verwendung nach einem der vorhergehenden Ansprüche, wobei der Promotor vorzugsweise aus der Gruppe ausgewählt ist, die aus Folgendem besteht: Cytomegalovirus (CMV)-Promotor, Phosphoglyceratkinase (PGK), CAG-Promotor (eine Kombination aus dem Cytomegalovirus-Early-Enhancer-Element und dem Hühner-Beta-Aktin-Promotor), saurem Gliafaserprotein (GFAP)-Promotor, Synapsin-1-Promotor, neuronenspezifischer Enolase (NSE) und induzierbaren Promotoren wie zum Beispiel Gene Switch oder ein vom Tet-Operon abgeleiteter Promotor.

9. AAV-Gentherapievektor zur Verwendung nach einem der vorhergehenden Ansprüche, wobei der Gentherapievektor ferner vor, gleichzeitig oder nach der intrathekalen lumbalen Verabreichung in die Cisterna magna verabreicht wird.

10. AAV-Gentherapievektor zur Verwendung nach einem der vorhergehenden Ansprüche, wobei dem Patienten 1 × 10¹⁴ - 2 × 10¹⁵ Genomkopien pro Kilogramm Körpergewicht verabreicht werden.

11. AAV-Gentherapievektor zur Verwendung nach Anspruch 10, wobei dem Patienten 1 × 10¹⁴ - 6 × 10¹⁴ Genomkopien pro Kilogramm Körpergewicht verabreicht werden.

12. AAV-Gentherapievektor zur Verwendung nach einem der vorhergehenden Ansprüche, wobei der Patient keiner Vorbehandlung mit einer Substanz unterzogen wird, die den intrakraniellen Druck kontrolliert und/oder die interzellulären engen Verbindungen zwischen Endothelzellen der ZNS-Mikrovaskulatur unterbrechen kann und/oder die Abgabe von Medikamenten, wie zum Beispiel Chemotherapeutika, an das ZNS erleichtert.

13. AAV-Gentherapievektor zur Verwendung nach einem der vorhergehenden Ansprüche, wobei der Patient vor der intrathekalen lumbalen Verabreichung des AAV-Gentherapievektors keiner intravenösen Vorbehandlung mit Mannitol unterzogen wird.

14. AAV-Gentherapievektor zur Verwendung nach einem der vorhergehenden Ansprüche, wobei der AAV-Gentherapievektor kein selbstkomplementärer Genvektor ist.

## Revendications

1. Vecteur de thérapie génique de virus adéno-associé (AAV) pour l'utilisation comme médicament pour traiter des troubles du SNC chez un sujet mammifère, où le sujet mammifère est un humain, où le vecteur de thérapie génique comprend des protéines de capside d' AAV de sérotype 5 et un produit génique d'intérêt flanqué de répétitions terminales inversées (ITR) d'AAV et où le produit génique d'intérêt est lié de manière opérationnelle à des éléments de contrôle d'expression comprenant un promoteur qui produit une expression suffisante du produit génique d'intérêt pour obtenir un effet thérapeutique, et où le vecteur de thérapie génique est administré par administration intrathécale lombaire, où 1 × 10¹⁴ - 1 × 10¹⁶ copies de génome par kilogramme de poids corporel sont administrées au sujet.

2. Vecteur de thérapie génique AAV pour l'utilisation selon la revendication 1, où l'administration intrathécale lombaire se fait à une position sélectionnée dans le groupe constitué par L4 - L5, L3 - L4, L1 - L2 et L2 - L3.

3. Vecteur de thérapie génique AAV pour l'utilisation selon la revendication 1 ou la revendication 2, où le vecteur de thérapie génique AAV est un vecteur de thérapie génique AAV simple brin ou un vecteur duplex monomérique.

4. Vecteur de thérapie génique AAV pour l'utilisation selon l'une quelconque des revendications précédentes, où le produit génique d'intérêt est pour l'utilisation dans le traitement ou la prophylaxie d'une affection sélectionnée dans le groupe constitué par : la sclérose latérale amyotrophique (SLA), l'amyotrophie spinale (SMA), la douleur, la maladie d'Huntington, la maladie d'Alzheimer, la maladie de Tay-Sachs, l'ataxie de Friedreich, l'ataxie télangiectasie, l'ataxie spinocérébelleuse de type 1, 2 et 3, la maladie de Niemann-Pick A, B et C, la dystonie dopa-sensible, le syndrome de l'X fragile, la maladie de Krabbe, la maladie de stockage du glycogène de type 2 (Pompe), la sclérose latérale primaire, la maladie de Pelizaeus-Merzbacher, l'adrénoleucodystrophie liée à l'X, la neuropathie axonale géante, l'atrophie multisystématisée (MSA), la myopathie myotonique proximale, la céroïde neuronale lipofuscinose (maladie de Batten) et le cancer.

5. Vecteur de thérapie génique AAV pour l'utilisation selon l'une quelconque des revendications précédentes, où les ITR d'AAV sont des ITR d'AAV de sérotype 2.

6. Vecteur de thérapie génique AAV pour l'utilisation selon l'une quelconque des revendications précédentes, où le produit génique d'intérêt est sélectionné dans le groupe constitué par : l'aspartylglucosaminidase, l'α-galactosidase A, la palmitoyl-protéine thioestérase, la tripeptidyl-peptidase, la protéine transmembranaire lysosomale, des produits géniques multiples, le transporteur de cystéine, la céramidase acide, l'α-L-fucosidase acide, la protéine protectrice/cathepsine A, la β-glucosidase acide ou la glucocérébrosidase, la β-galactosidase acide, l'iduronate-2-sulfatase, l'α-L-iduronidase, la galactocérébrosidase, l'α-mannosidase acide, la β-mannosidase acide, l'arylsulfatase B, l'arylsulfatase A, la N-acétylgalactosamine-6-sulfate sulfatase, la β-galactosidase acide, la N-acétylglucosamine-I-phosphotransférase, la sphingomyélinase acide, le NPC-1, l'α-glucosidase acide, la β-hexosaminidase B, la N-sulfatase héparane, l'α-N-acétylglucosaminidase, l'acétyl-CoA : α-glucosaminide N-acétyltransférase, la N-acétylglucosamine-6-sulfate sulfatase, l'α-N-acétylgalactosaminidase, l'α-N-acétylgalactosaminidase, l'α-neuramidase, la β-glucuronidase, la β-hexosaminidase A, la lipase acide, des facteurs neurotrophiques tels que le facteur de croissance des nerfs (NGF), la neurotrophine-3 (NT-3), la neurotrophine-4/5 (NT-4/5), le facteur neurotrophique dérivé du cerveau (BDNF), le facteur neurotrophique dopaminergique cérébral (CDNF), le facteur neurotrophique dérivé de la lignée cellulaire gliale (GDNF), le facteur neurotrophique ciliaire (CNTF), le facteur de croissance analogue à l'insuline (IGF-1) et le miRNA pour la régulation à la baisse d'un gène défectueux.

7. Vecteur de thérapie génique AAV pour l'utilisation selon l'une quelconque des revendications précédentes, où le produit génique d'intérêt est pour l'utilisation dans le traitement ou la prophylaxie d'une affection associée aux motoneurones et/ou aux neurones des ganglions de la racine dorsale (DRG).

8. Vecteur de thérapie génique AAV pour l'utilisation selon l'une quelconque des revendications précédentes, où le promoteur est de préférence sélectionné dans le groupe constitué par : le promoteur du cytomégalovirus (CMV), la phosphoglycérate kinase (PGK), le promoteur CAG (une combinaison de l'élément activateur précoce du cytomégalovirus et du promoteur de la bêta-actine de poulet), le promoteur de la protéine acide fibrillaire gliale (GFAP), le promoteur de la synapsine-1, l'énolase spécifique des neurones (NSE) et les promoteurs inductibles tels que Gene Switch ou un promoteur dérivé de l'opéron tet.

9. Vecteur de thérapie génique AAV pour l'utilisation selon l'une quelconque des revendications précédentes, où le vecteur de thérapie génique est en outre administré dans la Cisterna magna avant, simultanément ou après l'administration intrathécale lombaire.

10. Vecteur de thérapie génique AAV pour l'utilisation selon l'une quelconque des revendications précédentes, où 1 × 10¹⁴ - 2 × 10¹⁵ copies de génome par kilogramme de poids corporel sont administrées au sujet.

11. Vecteur de thérapie génique AAV pour l'utilisation selon la revendication 10, où 1 × 10¹⁴ - 6 × 10¹⁴ copies de génome par kilogramme de poids corporel sont administrées au sujet.

12. Vecteur de thérapie génique AAV pour l'utilisation selon l'une quelconque des revendications précédentes, où le sujet n'est pas soumis à un prétraitement avec une substance qui contrôle la pression intracrânienne et/ou peut perturber les jonctions serrées intercellulaires entre les cellules endothéliales de la microvasculature du SNC et/ou facilite l'administration de médicaments tels que des chimiothérapies au SNC.

13. Vecteur de thérapie génique AAV pour l'utilisation selon l'une quelconque des revendications précédentes, où le sujet n'est pas soumis à un prétraitement par mannitol intraveineux avant l'administration intrathécale lombaire du vecteur de thérapie génique AAV.

14. Vecteur de thérapie génique AAV pour l'utilisation selon l'une quelconque des revendications précédentes, où le vecteur de thérapie génique AAV n'est pas un vecteur génétique auto-complémentaire.
